# EUROPEAN PATENT SPECIFICATION

(11) **EP 1 549 673 B1**
(45) Date of publication and mention of the grant of the patent: **30.07.2008**
(21) Application number: 03748078.7
(22) Date of filing: 24.09.2003
(51) Int. Cl.: C07K 14/47, C12N 15/00, C07K 16/18, A61K 38/17, A61K 39/395

(54) **NOVEL MHC II ASSOCIATED PEPTIDES**
NEUE, MHC KLASSE II ASSOZIIERTE PEPTIDE
NOUVEAUX PEPTIDES ASSOCIES AU MHC II

(30) Priority: 02.10.2002 EP 02022224
(43) Date of publication of application: 06.07.2005
(73) Proprietor: F. Hoffmann-La Roche AG, 4002 Basel (CH)
(72) Inventor: KROPSHOFER, Harald, 79539 Loerrach (DE); ROEHN, Till, Alexander, 8047 Zürich (CH); VOGT, Anne, 79539 Loerrach (DE)
(74) Representative: Witte, Hubert
(86) International application number: PCT/EP2003/010602
(87) International publication number: WO 2004/031230

(56) References cited:
- EP-A- 1 120 651
- ADEM C ET AL: "Wide spectrum screening keratin as a marker of metaplastic spindle cell carcinoma of the breast: An immunohistochemical study of 24 patients" HISTOPATHOLOGY (OXFORD), vol. 40, no. 6, June 2002 (2002-06), pages 556-562, XP002266245 ISSN: 0309-0167
- DE ARAUJO V C ET AL: "Salivary duct carcinoma: Cytokeratin 14 as a marker of in-situ intraductal growth" HISTOPATHOLOGY (OXFORD), vol. 41, no. 3, September 2002 (2002-09), pages 244-249, XP002266246 ISSN: 0309-0167
- PRODUCT DATA SHEET: "Monoclonal Anti-Vimentin Clone LN-6" 1989, SIGMA , XP002266247 * the whole document *
- PRODUCT DATA SHEET: "Monoclonal anti-vimentin clone VIM 13.2" 1998, SIGMA , XP002266248 * the whole document *
- WANG R-F ET AL: "HUMAN TUMOR ANTIGENS FOR CANCER VACCINE DEVELOPMENT" IMMUNOLOGICAL REVIEWS, MUNKSGAARD, XX, vol. 170, 1999, pages 85-100, XP001015479 ISSN: 0105-2896
- TOCKMAN M ET AL: "QUANTITATION OF MOLECULAR MARKERS FOR PRE-NEOPLASTIC LUNG CANCER" AMERICAN REVIEW OF RESPIRATORY DISEASE, NEW YORK, NY, US, vol. 143, no. 4 PART 2, 1991, page A203, XP009029202 ISSN: 0003-0805
- PASTORINO U ET AL: "IMMUNOCYTOCHEMICAL MARKERS IN STAGE I LUNG CANCER: RELEVANCE TO PROGNOSIS" JOURNAL OF CLINICAL ONCOLOGY, GRUNE AND STRATTON, NEW YORK, NY, US, vol. 15, no. 8, August 1997 (1997-08), pages 2858-2865, XP009007844 ISSN: 0732-183X
- HANASH S ET AL: "A PROTEOMIC APPROACH TO THE IDENTIFICATION OF LUNG CANCER MARKERS" DISEASE MARKERS, WILEY, CHICHESTER, GB, vol. 17, no. 4, 2001, pages 295-300, XP008026267 ISSN: 0278-0240

## Description

The present invention relates to the identification of novel tumor antigenic peptides bound to human MHC class II HLA-DR molecules. This invention relates to the presentation of such tumor antigenic peptides by dendritic cells after engagement of tumor cells. Moreover, the invention relates also to the use of such tumor antigenic peptides for vaccination against tumors as well as for diagnosis of immune responses against tumors.

Tumor cells can be distinguished from healthy cells by the ex-pression of tumor-specific proteins. These proteins which are newly expressed, mutated or aberrantly expressed in tumors can be utilized as diagnostic markers or for therapy.

A potent class of markers serving as both diagnostic and therapeutic tools, are protein fragments or peptides bound to molecules of the major histocompatibility complex (MHC). In humans, MHC molecules are termed human leukocyte antigens (HLA). HLA-associated peptides are short, encompassing 9-25 amino acids (Kropshofer, H. & Vogt, A.B., Immunol Today 18 (1997) 77-82). They are indispensable for mounting an adaptive immune response as they activate specialized immune cells, named T lymphocytes (short: T cells). The lack of T cell recognition of peptides derived from tumor-specific antigens contributes to immune evasion and progressive growth of tumors (Boon, T. et al., Ann Rev Immunol. 12 (1994) 337-265).

With regard to their function, two classes of MHC-peptide complexes can be distinguished (Germain; R., Cell 76 (1994) 287-299): (i) MHC class I-peptide complexes can be expressed by almost all nucleated cells in order to attract CD8+ cytotoxic T cells which lyse tumor cells and infected cells, (ii) MHC class II-peptide complexes are constitutively expressed only on so-called antigen presenting cells (APCs), such as B lymphocytes, macrophages or dendritic cells (DCs). In particular, DCs have the capacity to prime CD4+ T helper cells (Banchereau, J. & Steinman, R.M., Nature 392 (1998) 245-254). Moreover, DCs can be licensed to optimally activate cytotoxic CD8+ T cells: this is accomplished through prior interaction of their MHC class II-peptide complexes with CD4+ T helper cells (Ridge, T. et al., Nature 393 (1998) 474-478). Thus, peptides presented by MHC class II molecules on DCs play a superior role in the pathogenesis of diseases involving T cell-driven immune responses, hence also in the induction of immunity against tumors.

The apparent role of DCs in initiating immune responses has stimulated attempts to exploit DCs as vaccines, in particular against cancer (Dallal, R.M. & Lotze, M.T., Curr Opinion Immunol 12 (2000) 583-588). A key advance was the invention of techniques for differentiation of DCs *in vitro* from different sources including peripheral blood, e.g. adherent monocytes, or bone marrow-derived CD34+ stem-cell precursors. DCs differentiated and activated *in vitro* can be used for vaccination of cancer patients after co-culture with tumor cell-derived antigens or by employing analogous techniques. Pilot dendritic cell vaccination studies have successfully induced specific anticancer responses including clinical responses (Timmermann, J.M. & Levy, R., Ann Rev Medicine 50 (1999) 507-529; Nestle, F.O., et al., Nature Medicine 7 (2001) 761-765).

Vaccines based on the identification of tumor antigens include DCs primed with naked DNA, recombinant adeno- or vaccinia viruses, natural or recombinant proteins purified from the respective tumor cells or synthetic analogs of tumor peptides. The advantage of pulsing DCs with antigenic tumor peptides rather than with genetic or protein precursors is that peptides can directly be loaded onto MHC molecules of DCs without further processing.

During the past decade, numerous peptides derived from tumor marker proteins and restricted by MHC class I molecules have been identified. They are grouped into four categories: cancer-testes antigens, melanoma-melanocyte differentiation antigens, mutated antigens and non-mutated shared antigens over-expressed on tumors. In several clinical pilot vaccination studies, DCs from melanoma patients were pulsed with cocktails of melanoma peptides which, as yet, were exclusively HLA class I-restricted (Nestle, F.O. et al., Nature Medicine 4 (1998) 328-332; Thurner, B. et al., J Exp Med 190 (1999) 1669-1678). However, there is increasing evidence that the efficacy and longevity of cytotoxic T cell responses against tumors can be increased by the recruitment of MHC class II -restricted helper T cells. Hence, an improved vaccination method would foresee the combinatorial use of MHC class II associated tumor peptides in addition to MHC class I antigens.

Knowledge of MHC class II-restricted cancer antigens recognized by CD4+ T helper cells lags behind the identification of class I-restricted antigens (Wang, R.-F., Trends in Immunol 22 (2001) 269-276). One reason is that transfection of cDNA libraries from tumor cells into target cells followed by usage of anti-tumor T cells to identify the appropriate transfectants and antigenic epitopes - a method successfully employed with MHC class I molecules - is not effective because the encoded proteins do not travel to the MHC class II pathway in APCs.

An innovative alternative is to use autologous DCs pulsed with tumor cells or particular tumor marker proteins and sequence the peptides associated to MHC on DCs. This approach, however, has only be empolyed for vaccination against autologous tumors but, so far, not for identification of candidate tumor antigens, since DCs are non-dividing cells in vitro and only available in very small amounts from peripheral blood or bone marrow. Moreover, peptide purification and sequencing techniques were by far too insensitive, as yet, to directly identify disease-associated peptides by this approach or any other approach focusing on peptides generated in the human body.

Hence, the problem posed by the lack of knowledge of MHC class II restricted tumor antigenic peptides is solved by providing novel naturally-processed MHC class II associated candidate tumor antigenic peptides.

The present invention provides novel naturally-processed antigenic peptides which are candidate tumor antigens in melanoma and other tumors. These antigenic peptides are presented by human MHC class II HLA-DR molecules. They originate from the translation factor eIF-4A, the IFN-gamma-inducible protein p78, the cytoskeletal protein vimentin and the iron-binding surface protein melanotransferrin. The antigenic peptides of the present invention can be used as markers in diagnosis of the respective tumors and in therapy as anti-tumor vaccines.

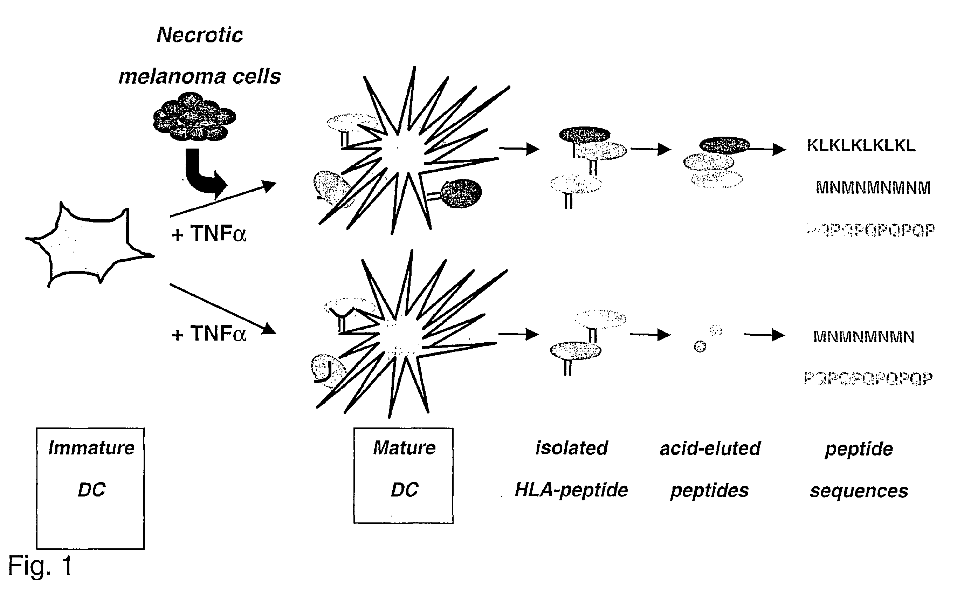

Fig. 1 is a diagram illustrating the technology used: Dendritic cells (DCs), the most specialized antigen presenting cells (APCs), are brought in contact with an antigenic source (e.g. necrotic melanoma cell) under optimal conditions for antigen uptake and antigen processing. As a control, DCs are cultured under the same conditions in the absence of melanoma cell antigens. After maturation of DCs antigen-loaded MHC class II molecules are purified and the respective MHC class II associated antigenic peptides isolated and identified.

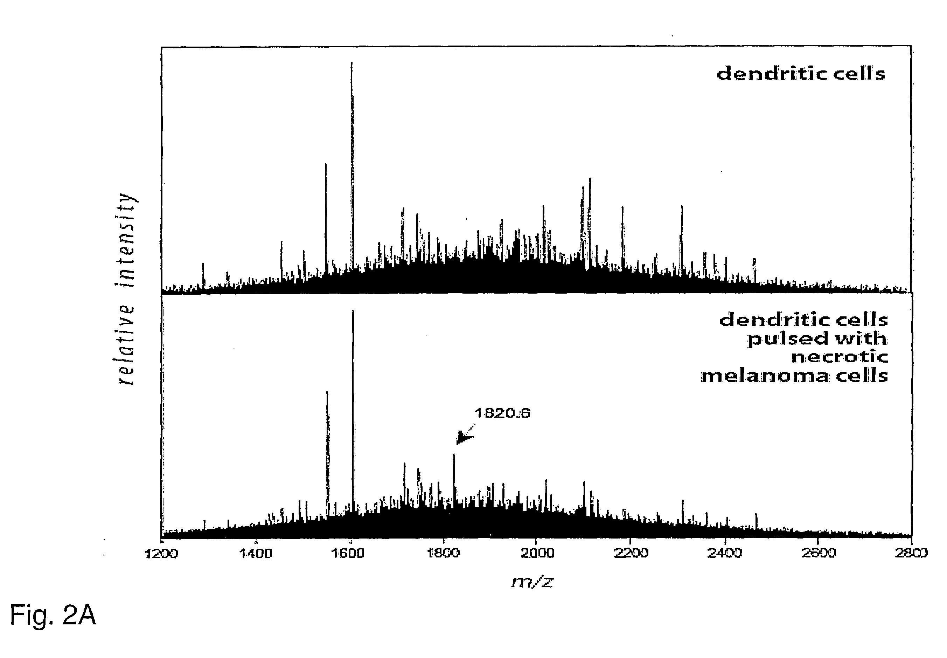

Fig. 2A contains a representative MALDI-mass spectrometric analysis of the repertoire of HLA-DR bound peptides isolated from mature dendritic cells which have been mock treated (upper panel) or pulsed with the necrotic melanoma cell line UKRV-Mel-15a (lower panel). Marked is the peptide peak (M+H⁺)=1820.6 which became dominant in the profile upon contact with melanoma cells.

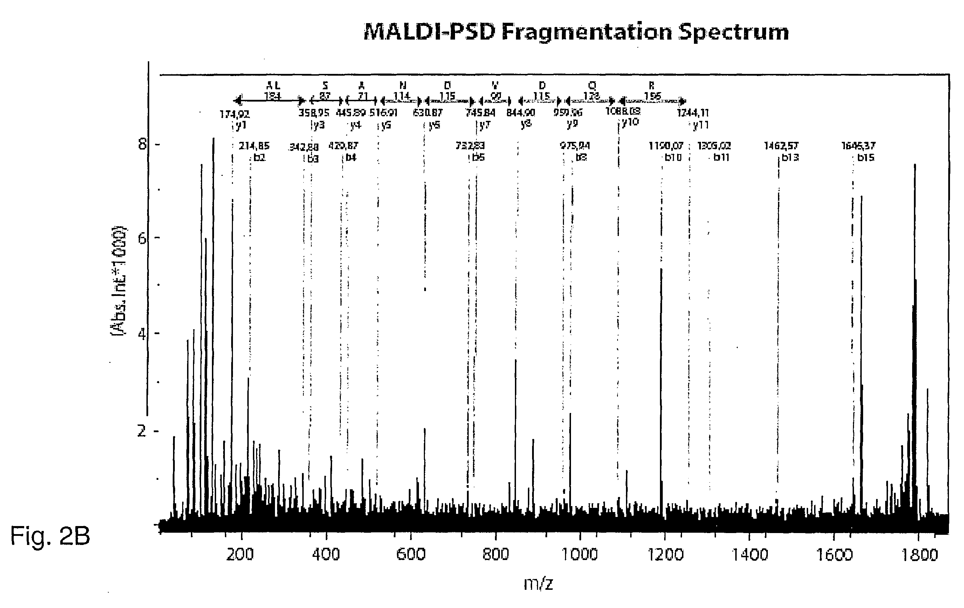

Fig. 2B shows the corresponding MALDI-PSD fragmentation spectrum of the peptide with the experimental mass (M+H⁺)=1820.6. This peptide was induced by necrotic melanoma cells (Fig. 2A). Data base search led to the identification of the vimentin epitope vimentin(202-217) (cf. Table 1).

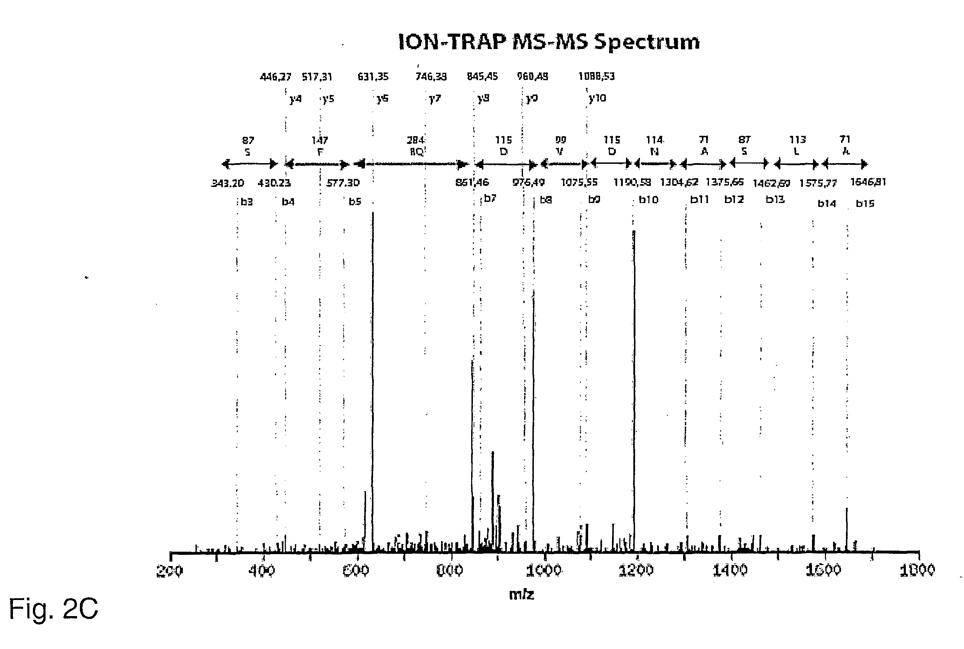

Fig. 2C shows an Ion trap MS-MS spectrum of a peptide with the experimental mass (M+H⁺)=1820.6. This peptide was induced by necrotic melanoma cells (Fig. 2A). Data base search led to the identification of the vimentin epitope vimentin(202-217) (cf. Table 1).

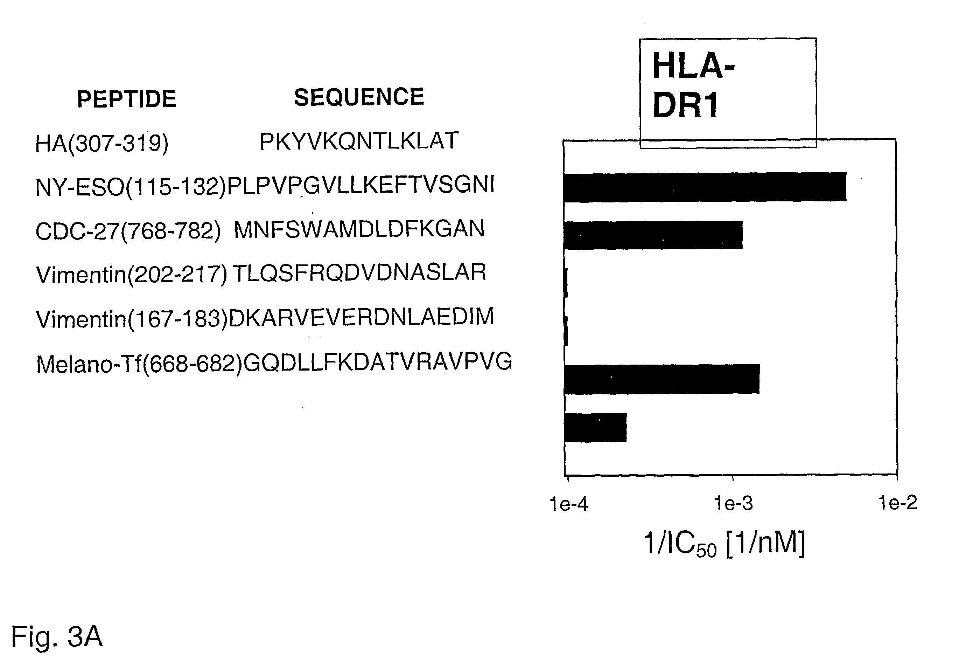

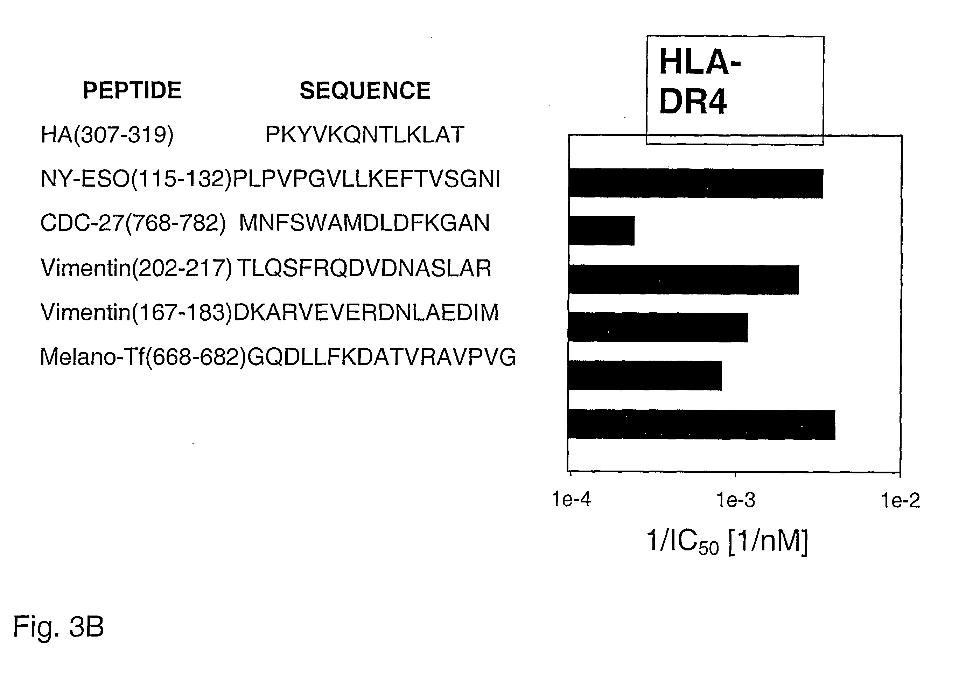

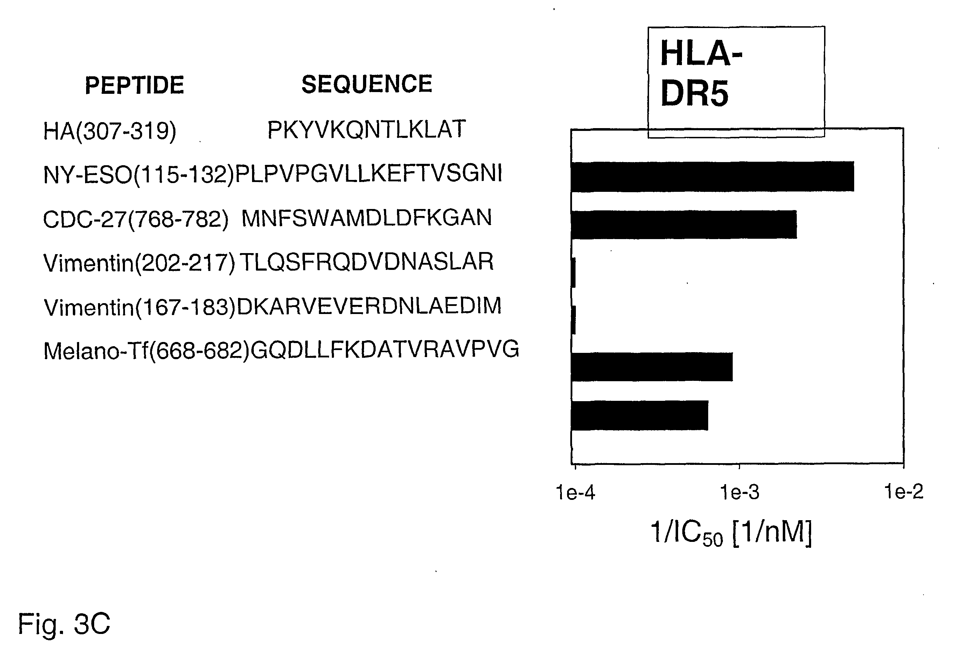

Fig. 3A-C shows the differential binding capacity of candidate tumor antigens derived from vimentin and melanotransferrin in the context of various HLA-DR allelic products. The indicated peptides were analyzed in an *in vitro* peptide-binding assay involving purified HLA-DR molecules and biotinylated HA(307-319) peptide as a reporter. As an affinity measure, the peptide concentration needed to reduce binding of biotinylated influenza virus hemagglutin HA(307-319) peptide by 50% (IC₅₀) through competition was determined. The reciprocal, 1/IC₅₀ is given, which directly correlates with the peptide affinity. For comparison, HA(307-319) from influenza virus hemagglutin, the tumor antigen NY-ESO(115-132) which is known to bind promiscuously to several HLA-DR alleles (Zarour HM et al., Cancer Research 2002; 62,213-218) and CDC-27(768-782) (cf. Table 2) have been included. The IC₅₀ values have been determined in the context of HLA-DR1 (Fig. 3A), HLA-DR4 (Fig. 3B) and HLA-DR5 (Fig. 3C).

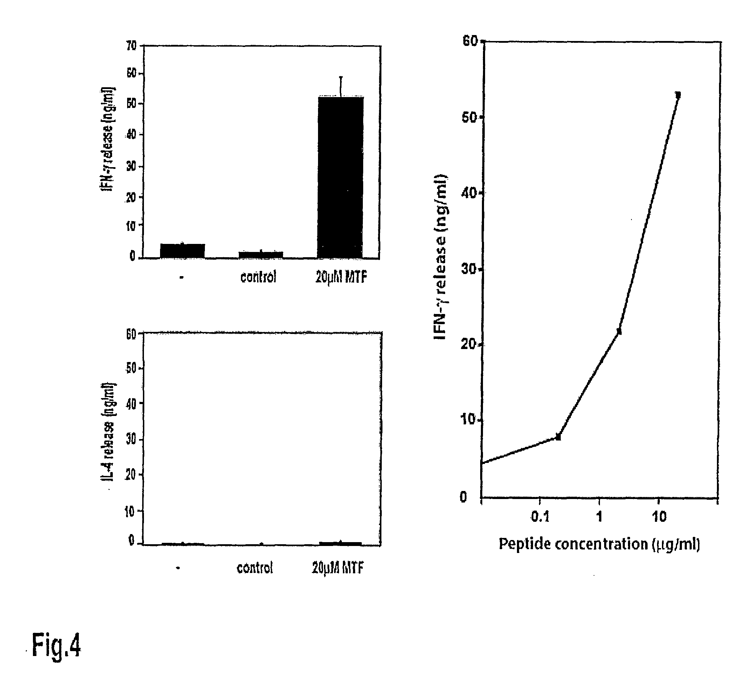

Fig. 4 shows the specific response of a T cell line generated against the identified epitope derived from melanotransferrin. Autologous dendritic cells were activated with LPS (1µg/ml) and pulsed with 20µM of a control peptide derived from the invariant chain (LPKPPKPVSKMRMATPLLMQALPM; SEQ ID NO: 17) or the melanotransferrin peptide (MTF; SEQ ID NO: 13) for 24h or were left unpulsed. T-cell responses were measured by sandwich immunoassays for INF-γ (TH1 response) or IL-4 (TH2 response).

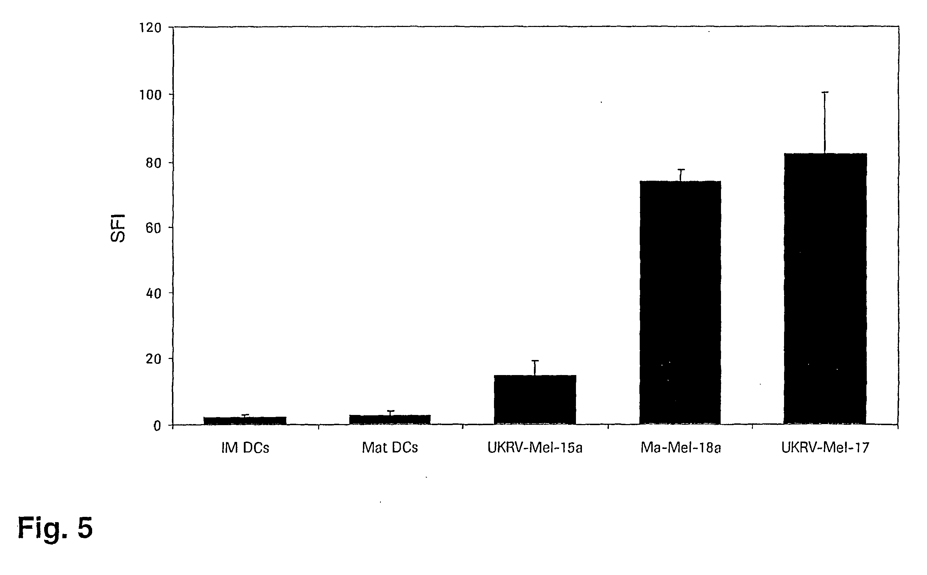

Fig. 5 shows the cell surface expression of melanotransferrin protein on a panel of melanoma cells (UKRV -Mel-15a, Ma-Mel-18a, UKRV -Mel-17 (Eichmuller S, Usener D, Jochim A, Schadendorf D., Exp Dermatol. 2002 Aug;ll(4):292-301)) as well as immature (IM DCs) and mature dendritic cells (Mat DCs) (activated with 1µg/ml LPS). The melanotransferrin mAb L235 (5µg/ml) was used for staining of the cells. The intensity of cell surface expression is specified as specific fluorescence index (SFI) i.d. geo mean of specific signal/ geo mean of isotype control.

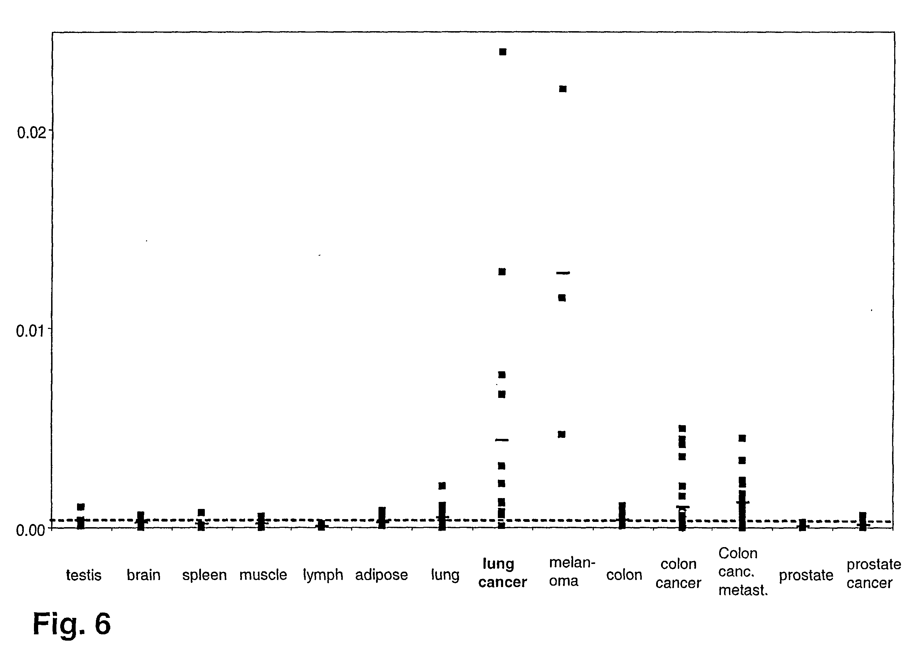

Fig. 6 shows the single target expression profiling (STEP) for melanotransferrin in different cancer vs. normal tissues. The expression level is given in arbitrary units based on the relative expression of melanotransferrin mRNA to a panel of 8 housekeeping genes. The dotted line shows the average expression of melanotransferrin in all normal tissues that were assessed.

Only a few human tumor antigens presented by MHC class II molecules have been described so far, with nearly all of them being associated to malignant melanoma. The first melanoma antigenic peptides found were derived from the melanocyte-specific enzyme tyrosinase and restricted by HLA-DR4 (Topalian SL et al., PNAS 1994; 91, 9461-9465). Further 3 melanoma epitopes were found to originate from the MAGE family of proteins and presented by HLA-DR11 and HLA-DR13 (Manici S et al., J Exp Med 1999; 189, 871-876). Another set of melanoma antigens, known to contain also MHC class I tumor antigens, comprises Melan-A/MART-1 (Zarour HM et al., PNAS 2000; 97, 400-405), gp100 and annexin II (Li K et al. Cancer Immunol Immunother 1998; 47, 32-38). They all were shown to activate CD4+ T cells derived from melanoma patients in a HLA-DR4-restricted manner.

Only 3 MHC class II associated melanoma antigens rely on mutations: the HLA-DR1-restricted peptide from the glycolytic enzyme triose phosphate isomerase (Pieper R et al., J Exp Med 1999; 189, 757-765), the HLA-DR4-restricted epitope from the cell cycle regulator CDC-27 (Wang R-F et al. J Exp Med 1999; 183, 1131-1140) and a melanoma epiotpe which relies on a chromosomally rearranged fusion protein composed of the LDL receptor and fucosal transferase (Wang R-F et al., J Exp Med 1999; 189, 1659-1667).

The term melanoma includes, but is not limited to, melanomas, metastatic melanomas, melanomas derived from either melanocytes or melanocyte related nevus cells, melanocarcinomas, melanoepitheliomas, melanosarcomas, melanoma in situ, superficial spreading melanoma, nodular melanoma, lentigo maligna melanoma, acral lentiginous melanoma, invasive melanoma or familial atypical mole and melanoma (FAM-M) syndrome. Such melanomas in mammals may be caused by, chromosomal abnormalities, degenerative growth and developmental disorders, mitogenic agents, ultraviolet radiation (UV), viral infections, inappropriate tissue expression of a gene, alterations in expression of a gene, and presentation on a cell, or carcinogenic agents.

The antigenic peptides of the invention are peptides, which are associated with and presented by MHC molecules and thereby can have the potential to activate or tolerize T cells. Antigenic peptides presented by MHC class II molecules are therefore MHC class II associated or MHC class II antigenic peptides, whereas antigenic peptides presented by MHC class I molecules are MHC class I associated or MHC class I antigenic peptides.

Peptides which are derived from proteins that are encoded in the genome of the body or an APC are denoted as "self-peptides". The main function of self-peptides presented by DCs in the peripheral lymphoid organs is thought to be the induction of T cell tolerance against self-proteins.

Peptides derived from proteins encoded in the genome of bacteria, viruses or other foreign invaders and which differ from self-proteins are called "foreign antigenic." or "foreign" peptides. They are able to elicit a T cell response against foreign proteins they are derived from.

Tumor antigens are proteins expressed by tumor cells which give rise to T cells with anti-tumor reactivity. Tumor antigenic peptides are derived from such tumor antigens and, therefore, have the potential to activate tumor-reactive T cells.

Disclosed herein are isolated MHC class II associated antigenic peptides comprising an amino acid sequence selected from the group consisting of SEQ ID NOs. 1, 2, 3, 4, 5, 6, 7, 8, 9, 10, 11, 12, 13 and 21. The antigenic peptides have a length of less than 26 amino acids, more preferably a length of 11 to 25 amino acids. Preferred are the antigenic peptides of the invention with a length of 14 to 18 amino acids. Most preferred are the antigenic peptides of the invention with a length of 15 to 17 amino acids.

The MHC class II associated novel antigenic peptides originate from the cytoskeletal protein vimentin (SEQ ID NOs. 1 to 6), the translation faction eIF-4A1 (SEQ ID NOs. 7 to 9), the IFN-γ inducible protein p78 (SEQ ID NOs. 10 and 11) and the iron-binding surface protein melanotransferrin (SEQ ID NOs. 12 and 13) and melanoma antigen recognized by T-cells 1 (MART-1, Melan-A protein; SEQ ID NO: 21).

The single peptide binding groove of MHC class II molecules is about 25 Å long, but in contrast to MHC class I molecules, both sides are open (Stern LJ et al., Nature 1994; 368, 215-221). Thus, naturally processed antigenic peptides eluted from human MHC class II molecules have a minimal length of about 11 residues and attain a maximal length of about 25 residues (Chicz RM et al., J Exp Med 1993; 178, 27-47).

The stability of the MHC-peptide interaction is determined by more than a dozen hydrogen bonds involving the peptide backbone and the complementarity between specificity pockets of the binding groove and appropriately located amino acid side-chains of the peptide. The amino acids of the peptide fitting into the respective pockets were names "anchor" residues. With regard to most HLA-DR alleles, these anchors are located at relative positions P1, P4, P6 and P9. The combination of amino acids at these 4 anchor positions conferring high-stability binding to the respective HLA-DR allelic product and vary from allele to allele. The peptide binding motif is defined herein as the sequence of nine amino acids comprising the four anchor amino acids. The peptide binding motif of the MHC class II antigenic peptide is depicted in SEQ ID NO. 18 for the antigenic peptide of SEQ ID NOs. 1 to 4, or in SEQ ID NO. 19 for the antigenic peptide of SEQ ID NOs. 5 and 6 derived from vimentin, and in SEQ ID NO. 20 for the antigenic peptide of SEQ ID NOs. 12 and 13 derived from melanotransferrin.

Additional binding energy is provided by hydrogen bonds involving residues in front of the P1 anchor and behind the P9 anchor. In agreement with that, in most naturally processed peptides the nonameric core-region (P1-P9) is N- and C-terminally flanked by 3-4 residues. Hence, the majority of peptides are 15-17-mers. Longer peptides protrude from the groove, thereby allowing access of exopeptidases which are trimming both ends.

The antigenic peptides comprising an amino acid sequence selected from the group consisting of SEQ ID NOs. 1, 2, 3, 4, 5, 6, 7, 8, 9, 10, 11, 12, 13 and 21 may have amino acid deletions at the amino or carboxy terminus and maintain their binding capacity. The relative binding capacity of a peptide is measured by determining the concentration necessary to reduce binding of a labelled reporter peptide by 50%. This value is called IC₅₀. Peptide binding with a reasonable affinity to the relevant HLA class II molecules attain IC₅₀ values not exceeding 10-fold the IC₅₀ of established reference peptides. Most preferred is the antigenic peptide of the invention consisting of the peptide binding motif comprising the four anchor amino acids.

### Vimentin

Vimentin is known to be a marker protein in a variety of benign and malign tumors. Together with melanA/MART-1, tyrosinase and S100, vimentin is routinely used to trace melanoma cells in clinical specimens from melanoma patients. Interestingly, melanoma clones with low invasive potential have a high vimentin expression, whereas vimentin is downregulated in highly invasive melanoma cell clones (Gutgemann A et al., Arch Dermatol Research 2001; 293, 283-290). In contrast, enhanced expression of vimentin is observed in poorly differentiated and metastatic prostate carcinoma (Lang SH et al., Prostate 2002; 52, 253-263). Moreover, vimentin is overexpressed in human renal cell carcinoma in relation to normal renal tissue (Stassar MJ et al. Br. J. Cancer 2001; 85, 1372-1382). Likewise, >95% of tumor cells in classical Hodgkin's lymphoma are vimentin positive, whereas T-cell-rich B-cell lymphomas are negative for vimentin (Rudiger T et al., Am J Surg Path 1998, 22, 1184-91).

### eIF-4A1

In recent years, a strong relation between the activities of translation initiation factors and malignant cell transformation has been reported in a number of studies including breast carcinomas, neuroblastomas and melanomas (Kerekatte V et al., Int. J. Cancer 1995; 64, 27-31). These findings have led to the definition of a new group of translational oncogenes. The translation initiation factor eIF-4A1 is consistently overexpressed in melanoma cell lines in relation to normal human melanocytes. eIF-4A1 overexpression seems to be an important feature of melanoma cells and might contribute to their malignant transformation (Eberle J et al., Int. J. Cancer 1997; 71, 396-401).

### IFN-inducible p78

Prostate cancer progression from a hormon-dependent to a hormon-independent state includes a cascade of genetic alterations caused by activation of oncogenes and/ or inactivation of tumor suppressor genes. Several genes were identified which are highly overexpressed in androgen-independent cancer cell lines. Among other genes, the interferon-inducible gene encoding p78 was identified (Markku H et al., Lab Invest. 2000; 80, 1259-1268.).

### Melanotransferrin (p97)

Melanotransferrin was one of the first surface markers associated with human melanoma (Hellström et al., Int. J Cancer 1983; 31, 553-555). In contrast to the tumor marker proteins vimentin, eIF-4A1 or IFN-inducible p78, described above, melanotransferrin is expressed to a significant extent only in a few cell types: endothelial cells in liver and brain, the sweat gland ducts and neoplastic cells (Richardson DR, Eur J Biochem 2000; 267, 1290-1298). Besides tyrosinase, MUC 18 and Melan A/MART-1, melanotransferrin is routinely used as a gene marker in RT-PCR assays and found to be expressed in most human melanomas (Slingluff et al., Curr Opin Immunol 1994; 6, 733-740). Beyond that, melanotransferrin was shown to be up-regulated in glioblastoma, astrocytoma, meningioma and oligodendroglioma (Chi DD, et al., Am. J. Pathol. 1997; 150, 2143-2152). It is frequently found in liver, lung and kidney metastases of melanoma patients. Importantly, melanotransferrin is a GPI-anchored surface protein and, hence, accessible to antibodies.

### Melanoma antigen recognized by T-cells

Melanoma antigen recognized by T-cells (Melan A; MART-1) is a known tumor antigen (Kawakami Y., Eliyahu S., Delgado C.H., Robbins P.F., Rivoltini L., Topalian S.L., Miki T., Rosenberg S.A., Proc. Natl. Acad. Sci. U.S.A. 91:3515-3519(1994); Coulie P.G., Brichard V., van Pel A., Woelfel T., Schneider J., Traversari C., Mattei S., de Plaen E., Lurquin C., Szikora J.-P., Renauld J.-C., Boon T., J. Exp. Med. 180:35-42(1994)).

An "isolated" peptide of the invention is a peptide which either has no naturally-occurring counterpart (e.g., such as an mutated peptide antigen), or has been separated or purified from components which naturally accompany it, e.g., in tissues such as pancreas, liver, spleen, ovary, testis, muscle, joint tissue, neural tissue, gastrointestinal tissue, or body fluids such as blood, serum, or urine. Typically, the peptide is considered "isolated" when it is at least 70%, by dry weight, free from the proteins and naturally-occurring organic molecules with which it is naturally associated. Preferably, a preparation of a peptide of the invention consists of at least 80%, more preferably at least 90%, and most preferably at least 99%, by dry weight, the peptide of the invention. Since a peptide that is chemically synthesized is, by its nature, separated from the components that naturally accompany it, the synthetic peptide is "isolated." Immunogenic peptide includes, but is not limited to, an antigenic peptide capable of causing or stimulating a cellular or humoral immune response. Such peptides may also be reactive with antibodies.

The invention further provides analogs of the antigenic peptides of the invention. The term analog includes any peptide which displays the functional aspects of these antigenic peptides. The term analog also includes conservative substitutions or chemical derivatives of the peptides.

The term "analog" includes any polypeptide having an amino acid residue sequence substantially identical to the sequences described herein in which one or more residues have been conservatively substituted with a functionally similar residue and which displays the functional aspects of the peptides as described herein. Examples of conservative substitutions include the substitution of one non-polar (hydrophobic) residue such as phenylalanine, tyrosine, isoleucine, valine, leucine or methionine for another, the substitution of one polar (hydrophilic) residue for another such as between arginine and lysine, between glutamine and asparagine, between threonine and serine, the substitution of one basic residue such as lysine, arginine or histidine for another, or the substitution of one acidic residue, such as aspartic acid or glutamic acid for another.

The phrase "conservative substitution" also includes the use of a chemically derivatized amino acid in place of a non-derivatized amino acid. "Chemical derivative" refers to a subject polypeptide having one or more amino acids chemically derivatized by reaction of a functional side group. Examples of such derivatized molecules include for example, those molecules in which free amino groups have been derivatized to form amine hydrochlorides, p-toluene sulfonyl groups, carbobenzoxy groups, t-butyloxycarbonyl groups, chloroacetyl groups, acetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-imbenzylhistidine. Also included as chemical derivatives are those proteins or peptides which contain one or more naturally-occurring amino acid derivatives of the twenty standard amino acids. For examples: 4-hydroxyproline may be substituted for proline; 5-hydroxylysine may be substituted for lysine; 3-methylhistidine may be substituted for histidine; homoserine may be substituted for serine; and ornithine or citrulline may be substituted for lysine.

Therefore, in a preferred embodiment of the present invention, the isolated MHC class-II associated antigenic peptides with a length of less than 26 amino acids comprising an amino acid sequence selected from the group consisting of SEQ ID NOs. 12 and 13, as well as these peptides with deletions at the amino or carboxy terminus maintaining their binding capacity, contain at least one amino acid modification within their sequence to enhance binding of the peptide to a MHC class II molecule. The amino acid modification may be a conservative amino acid substitution as described above. The binding may be determined as the binding capacity compared to a reference antigenic peptide. The peptide binding motif of the MHC class II antigenic peptide may also comprise at least one, at least two, at least three, at least four or at least five modifications of the amino acid sequence while still attaining the binding capacity of the non-modified peptide binding motif. Preferably, the modified peptide binding motif comprises at least three of the four anchor amino acids of the non-modified peptide binding motif. The amino acid modification may be a conservative amino acid substitution as described above.

An isolated peptide of the invention can be obtained, for example, by extraction from a natural source (e.g., elution from MHC II molecules); by expression of a recombinant nucleic acid encoding the peptide; or by chemical synthesis. A peptide that is produced in a cellular system different from the source from which it naturally originates is "isolated," because it will be separated from components which naturally accompany it. The recombinant peptide expressed by a host organism can be obtained as a crude lysate or can be purified by standard protein purification procedures known in the art which may include differential precipitation, size exclusion chromatography, ionexchange chromatography, isoelectric focusing, gel electrophoresis, affinity, and immunoaffinity chromatography and the like. The extent of isolation or purity can be measured by any appropriate method, e.g. mass spectrometry or HPLC analysis. The peptides may be prepared synthetically by procedures described in Merrifield, (1986) Science 232: 341-347, and Barany and Merrifield, The Peptides, Gross and Meienhofer, eds (N. Y., Academic Press), pp. 1-284 (1979). The synthesis can be carried out in solution or in solid phase or with an automatized synthesizer (Stewart and Young, Solid Phase Peptide Synthesis, 2nd ed., Rockford III., Pierce Chemical Co. (1984)).

In a further embodiment, the antigenic peptides of the invention are provided comprising an amino acid sequence selected from the group consisting of SEQ ID NOs. 12 and 13, as well as these peptides with deletions at the amino or carboxy terminus maintaining their binding capacity, linked to a MHC class II molecule.

Multimers (e.g., dimers, trimers, tetramers, pentamers, hexamers or oligomers) of a class II MHC molecule containing a covalently or non-covalently bound peptide defined by the method of the invention, if conjugated with a detectable label (e.g., a fluorescent moiety, a radionuclide, or an enzyme that catalyzes a reaction resulting in a product that absorbs or emits light of a defined wavelength) can be used to quantify T cells from a subject (e.g., a human patient) bearing cell surface receptors that are specific for, and therefore will bind, such complexes. Relatively high numbers of such T cells are likely to be diagnostic of a relevant disease or an indication that the T cells are involved in immunity to the disease. In addition, continuous monitoring of the relative numbers of multimer-binding T cells can be useful in establishing the course of a disease or the efficacy of therapy. Such assays have been developed using tetramers of class I MHC molecules containing an HIV-1-derived or an influenza virus-15 derived peptide (Altman et al. (1996), Science 274:94-96; Ogg et al. (1998), Science 279:2103- 21061), and corresponding class II MHC multimers would be expected to be similarly useful. Such complexes could be produced by chemical cross-linking of purified class II MHC molecules assembled in the presence of a peptide of interest or by modification of already established recombinant techniques for the production of class II MHC molecules containing a single defined peptide (Kazono et al. (1994), Nature 369:151-154; Gauthier et al. (1998), Proc. Natl. Acad. Sci. U.S.A. 95:11828-118331). The class II MHC molecule monomers of such multimers can be native molecules composed of full-length alpha and beta chains. Alternatively, they can be molecules containing either the extracellular domains of the alpha and beta chains or the alpha and beta chain domains that form the "walls" and "floor" of the peptide-binding cleft.

Therefore, the invention also relates to antibodies, fragments or derivatives thereof, directed to and reactive with the above described peptides. The general methodology for producing antibodies is well known and is disclosed per example in Kohler and Milstein, 1975, Nature 256,494 or in J. G. R. Hurrel, Monoclonal Hybridoma Antibodies: Techniques and Applications, CRC Press Inc., Boco Raron, FL (1982). The antibodies can be polyclonal or, preferably, monoclonal, or antibody fragments like be F (ab') 2, Fab, Fv or scFv. The antibodies of the present invention may also be humanized (Merluzzi S. et al., (2000), Adv. Clin. Path., 4(2): 77-85) or human antibodies (Aujame L. et al., Hum. Antibodies, (1997), 8(4): 155-168).

The present invention also provides an isolated nucleic acid molecule encoding an MHC class II antigenic peptide comprising a sequence selected from the group consisting of SEQ ID NO. 12 and 13, as well as a nucleic acid molecule encoding such an antigenic peptide with deletions at the amino or carboxy terminus maintaining its binding capacity and a nucleic acid molecule encoding such an antigenic peptide, wherein the amino acid sequence contains at least one amino acid modification to enhance binding of the peptide to a MHC class II molecule. Preferably, the isolated nucleic acid molecule is a DNA molecule.

Furthermore, an isolated nucleic acid molecule is provided encoding an antigenic peptide of the invention linked to a MHC class II molecule, wherein the antigenic peptide comprises an amino acid sequence selected from the group consisting of SEQ ID NOs. 12 and 13.

This invention also provides a recombinant nucleic acid construct comprising all or part of the nucleic acid sequence encoding an antigenic peptide comprising a sequence selected from the group consisting of SEQ ID NO.12 and 13, or comprising a nucleic acid molecule encoding such an antigenic peptide with deletions at the amino or carboxy terminus maintaining its binding capacity, or comprising a nucleic acid molecule encoding such an antigenic peptide, wherein the amino acid sequence contains at least one amino acid modification to enhance binding of the peptide to a MHC class II molecule, operably linked to an expression vector. Expression vectors suitable for use in the present invention comprise at least one expression control element operably linked to the nucleic acid sequence encoding the antigenic peptide. The recombinant expression construct may be a DNA construct.

The expression control elements are inserted in the vector to control and regulate the expression of the nucleic acid sequence encoding the antigenic peptide of the invention. Examples of expression control elements include, but are not limited to, lac system, operator and promoter regions of phage lambda, yeast promoters and promoters derived from polyoma, adenovirus, retrovirus or SV40. Additional preferred or required operational elements include, but are not limited to, leader sequence, termination codons, polyadenylation signals and any other sequences necessary or preferred for the appropriate transcription and subsequent translation of the nucleic acid sequence in the host system. It will be understood by one skilled in the art that the correct combination of required or preferred expression control elements will depend on the host system chosen. It will further be understood that the expression vector should contain additional elements necessary for the transfer and subsequent replication of the expression vector containing the nucleic acid sequence in the host system. Examples of such elements include, but are not limited to, origins of replication and selectable markers. It will further be understood by one skilled in the art that such vectors are easily constructed using conventional methods (www.cellbio.com/protocols.html) or are commercially available.

Another aspect of this invention relates to a host organism or a host cell into which a recombinant nucleic acid construct comprising all or part of the nucleic acid sequence encoding an antigenic peptide selected from the group consisting of SEQ ID NO. 12 and 13, or a nucleic acid molecule encoding such an antigenic peptide with deletions at the amino or carboxy terminus maintaining its binding capacity, or a nucleic acid molecule encoding such an antigenic peptide, wherein the amino acid sequence contains at least one amino acid modification to enhance binding of the peptide to a MHC class II molecule, operably linked to an expression vector, has been inserted. The host cells transformed with the nucleic acid constructs encompassed by this invention include eukaryotes, such as animal, plant, insect and yeast cells and prokaryotes, such as E. coli. The means by which the nucleic acid construct carrying the nucleic acid sequence may be introduced into the cell include, but are not limited to, microinjection, electroporation, transduction, or transfection using DEAE-dextran, lipofection, calcium phosphate or other procedures known to one skilled in the art (Sambrook et al. (1989) in "Molecular Cloning. A Laboratory Manual", Cold Spring Harbor Press, Plainview, New York).

In a preferred embodiment, eukaryotic expression vectors that function in eukaryotic cells are used. Examples of such vectors include, but are not limited to, retroviral vectors, vaccinia virus vectors, adenovirus vectors, herpes virus vector, fowl pox virus vector, plasmids, or the baculovirus transfer vectors. Preferred eukaryotic cell lines include, but are not limited to, COS cells, CHO cells, HeLa cells, NIH/3T3 cells, 293 cells (ATCC# CRL15731), T2 cells, dendritic cells, monocytes or Epstein-15 Barr Virus transformed B cells.

The present invention further provides a method for producing a MHC class II antigenic peptide comprising an amino acid sequence selected from the group consisting of SEQ ID NO. 12 and 13, or such an antigenic peptide with deletions at the amino or carboxy terminus maintaining its binding capacity, or such an antigenic peptide, wherein the amino acid sequence contains at least one amino acid modification to enhance binding of the peptide to a MHC class II molecule, comprising the steps of culturing the host cell containing a recombinant nucleic acid construct as described above under conditions allowing expression of said peptide and recovering the peptide from the cells or the culture medium.

The isolated and identified antigenic peptide sequences of the invention may be validated by the MHC binding motif, the MHC binding capacity and by T cell recognition.

### MHC binding motif

Peptides associated to a particular MHC molecule (allelic variant) have common structural characteristics, denoted as binding motifs, necessary to form stable complexes with MHC molecules. Peptide ligands eluted from MHC class I molecules are relatively short, ranging from 8-11 amino acids. Moreover, 2 or 3 side chains of the peptide are relevant for binding. The position of the respective amino acid side chains varies with the HLA allele, most often two of these so-called "anchor" residues are located at positions 2 and 9. With respect to a particular anchor position, only 1 or 2 amino acids normally can function as anchor amino acids e.g. leucine or valine V at position 2 in the case of HLA-A2.

In the case of MHC class II molecules, the peptide length varies from 11 to 25 amino acids, as longer peptides can bind since both ends of the peptide binding groove are open. Most HLA class II molecules accommodate up to 4 anchor residues at relative positions P1, P4, P6 and P9 contained in a nonameric core region. This core region, however, can have variable distance from the N-terminus of the peptide. In the majority of cases, 2-4 N-terminal residues precede the core region. Hence, the P1 anchor residues is located at positions 3, 4 or 5 in most HLA class II associated peptides. Peptides eluted from HLA-DR class II molecules share a big hydrophobic P1 anchor, represented by tyrosine, phenylalanine, tryptophane, methionine, leucine, isoleucine or valine.

The position and the exact type of anchor residues constitute the peptide binding motif which is known for most of the frequently occurring HLA class II allelic products. A computer algorithm allowing motif validation in peptide sequences is "Tepitope", available by vaccinome (www.vaccinome.com).

### MHC binding capacity

Peptides identified by the method of the invention may be tested for their ability to bind to the appropriate MHC class II molecule by methods known in the art using, for example, isolated MHC class II molecules and synthetic peptides with amino acid sequences identical to those identified by the method of the invention (Kropshofer H et al., J. Exp. Med. 1992; 175, 1799-1803; Vogt AB et al., J. Immunol. 1994; 153, 1665-1673; Sloan VS et al., Nature 1995; 375, 802-806). Alternatively, a cellular binding assay using MHC class II expressing cell lines and biotinylated peptides can be used to verify the identified epitope (Arndt SO et al., EMBO J., 2000; 19, 1241-1251)

In both assays, the relative binding capacity of a peptide is measured by determining the concentration necessary to reduce binding of a labelled reporter peptide by 50%. This value is called IC₅₀. Peptide binding with a reasonable affinity to the relevant HLA class II molecules attain IC₅₀ values not exceeding 10-fold the IC₅₀ of established reference peptides.

The same binding assays can also be used to test the ability of peptides to bind to alternative class II MHC molecules, i.e., class II MHC molecules other than those from which they were eluted using the method of the invention. The diagnostic methods of the invention using such peptides and therapeutic methods of the invention, using either the peptides or peptides derived from them, can be applied to subjects expressing such alternative class II MHC molecules.

### T cell recognition

The epitope verification procedure may involve testing of peptides identified by the method of the invention for their ability to activate CD4+ T cell populations. Peptides with amino acid sequences either identical to those identified in the present invention or corresponding to a core sequence derived from a nested group of peptides identified in the present invention are synthesized. The synthetic peptides are then tested for their ability to activate CD4+ T cells from (a) test subjects expressing the MHC class II molecule of interest and having at least one symptom of the disease; and (b) control subjects expressing the MHC class II molecule of interest and having no symptoms of the disease. Additional control subjects can be those with symptoms of the disease and not expressing the MHC class II molecule of interest.

In some diseases (e.g., those with an autoimmune component) responsiveness in the CD4+ T cells of test subjects but not in CD4+ T cells of the control subjects described in (b) provides confirmatory evidence that the relevant peptide is an epitope that activates CD4+ T cells that can initiate, promote, or exacerbate the relevant disease. In other diseases (e.g., cancer or infectious diseases without an autoimmune component), a similar pattern of responsiveness and non-responsiveness to that described in the previous sentence would indicate that the relevant peptide is an epitope that activates CD4+ T cells that can mediate immunity to the disease or, at least, a decrease in the symptoms of the disease.

CD4+ T cell responses can be measured by a variety of *in vitro* methods known in the art. For example, whole peripheral blood mononuclear cells (PBMC) can be cultured with and without a candidate synthetic peptide and their proliferative responses measured by, e.g., incorporation of [³H]-thymidine into their DNA. That the proliferating T cells are CD4+ T cells can be tested by either eliminating CD4+ T cells from the PBMC prior to assay or by adding inhibitory antibodies that bind to the CD4+ molecule on the T cells, thereby inhibiting proliferation of the latter. In both cases, the proliferative response will be inhibited only if CD4+ T cells are the proliferating cells. Alternatively, CD4+ T cells can be purified from PBMC and tested for proliferative responses to the peptides in the presence of APC expressing the appropriate MHC class II molecule. Such APC can be B-lymphocytes, monocytes, macrophages, or dendritic cells, or whole PBMC. APC can also be immortalized cell lines derived from B-lymphocytes, monocytes, macrophages, or dendritic cells. The APC can endogenously express the MHC class II molecule of interest or they can express transfected polynucleotides encoding such molecules. In all cases the APC can, prior to the assay, be rendered non-proliferative by treatment with, e.g., ionizing radiation or mitomycin-C.

As an alternative to measuring cell proliferation, cytokine production by the CD4+ T cells can be measured by procedures known to those in art. Cytokines include, without limitation, interleukin-2 (IL-2), interferon-gamma (IFN-gamma), interleukin-4 (IL-4), TNF-alpha, interleukin-6 (IL-6), interleukin-10 (IL-10), interleukin-12 (IL-12) or TGF-beta. Assays to measure them include, without limitation, ELISA, and bio-assays in which cells responsive to the relevant cytokine are tested for responsiveness (e.g., proliferation) in the presence of a test sample.

Alternatively, cytokine production by CD4+ lymphocytes can be directly visualized by intracellular immunofluorescence staining and flow cytometry.

Moreover the isolated antigenic peptides described beforehand may be used in the diagnosis, prevention and treatment of a disease, preferably of cancer. Therefore, the present invention provides in a further embodiment the antigenic peptides of the invention for use in the diagnosis, prevention and treatment of a disease, preferably of cancer.

One aspect of the invention is a therapeutic purpose, wherein one or more of the identified melanoma peptides are used to vaccinate patients against cancer, preferably melanoma. To this end, the relevant peptides may be directly administered to the patient, in an amount sufficient for the peptides to bind to the MHC molecules, and provoke activation of T cells followed by T cell-mediated lysis of infected or cancer cells.

Alternatively, melanoma peptides may be utilized for the generation of vaccines based on DCs. In this case, autologous DCs derived from patients' monocytes may be pulsed with the relevant peptides or recombinant proteins containing the relevant peptide sequences. Particularly, in vaccination against melanoma, a combination of the MHC class II associated peptides of the present invention without or in combination with MHC class I-associated tumor antigenic peptides known in the art may be used to pulse autologous or allogeneic DCs of melanoma patients. Similarly, nucleic acid molecules which encode the relevant peptides may be incorporated into a vector in order to transfect tumor cells. These transfected tumor cells may be fused with DCs. In any of these cases, DCs presenting the relevant peptides in context of the appropriate MHC molecules will be administered to the tumor patient for triggering cellular and/or antibody-mediated immune responses against the tumor.

The class II restricted melanoma antigens of this invention, or analogs thereof may be used as a vaccine either prophylactically or therapeutically. When provided prophylactically the vaccine is provided in advance of any evidence of melanoma. The prophylactic administration of the Class II restricted melanoma antigen vaccine should serve to prevent or attenuate melanoma in a mammal. In a preferred embodiment mammals, preferably human, at high risk for melanoma are prophylactically treated with the vaccines of this invention. Examples of such mammals include, but are not limited to, humans with a family history of melanoma, humans with a history of atypical moles, humans with a history ofFAM-M syndrome or humans afflicted with melanoma previously resected and therefore at risk for reoccurrence. When provided therapeutically, the vaccine is provided to enhance the patient's own immune response to the tumor antigen present on the melanoma or metastatic melanoma. The vaccine, which acts as an immunogen, may be a cell, cell lysate from cells transfected with a recombinant expression vector, cell lysates from cells transfected with a recombinant expression vector encoding for the Class II restricted melanoma antigen, or a culture supernatant containing the expressed protein. Alternatively, the immunogen is a partially or substantially purified recombinant protein, peptide or analog thereof encoding for a Class II restricted melanoma antigen. The proteins or peptides may be conjugated with lipoprotein or administered in liposomal form or with adjuvant using conventional methodologies.

Therefore, the present invention provides a pharmaceutical composition containing an effective amount of the antigenic peptides comprising the sequences depicted in SEQ ID NOs 12 and 13, as well as these peptides with deletions at the amino or carboxy terminus maintaining their binding capacity, and an acceptable excipient, diluent or carrier. "Effective amount" herein means a sufficient amount to activate specific lymphocytes and induce an effective response against the tumor. Such an amount will depend on the peptide used, the administration, the severity of the disease to be treated and the general conditions of the patient and will usually range from 1 to 50 mg/ml, for example in case of peptides being loaded on dendritic cells.

An acceptable excipient, diluent or carrier may be phosphate buffered saline for *in vitro* studies and physiological salt solutions for *in vivo* applications.

In one embodiment, such compositions will be used for the preventive vaccination of patients with predisposition to neoplasias or in the therapeutical vaccination of neoplastic patients. "Vaccination" herein means both active immunization, i. e. the *in vivo* administration of the peptides to elicit an *in vivo* immune response directly in the patient, as in conventional vaccination protocols, for example against pathogens, and passive immunization, i. e. the use of the peptides to activate *in vitro* anti-tumor CD4+ cells or autologous or allogeneic dendritic cells, which are subsequently re-inoculated into the patient.

The techniques for the preparation and the use of vaccines are known to those skilled in the art and are described, per example, in Paul, Fundamental Immunology, Raven Press, New York (1989) or Cryz, S. J., Immunotherapy and Vaccines, VCH Verlagsgesellschaft (1991). Vaccines are conventionally prepared in the form of injectables, suspensions or solutions, but they can also be used in the form of solid preparations or liposomes. The immunogenic ingredients can be mixed with pharmacologically acceptable excipients, such as emulsifiers, buffering agents and adjuvants which increase the efficacy of the vaccine. The latter can be administered according to single or multiple dosage schedules. Multiple dose provides 1 to 10 separate doses, each containing a quantity of antigen varying from 1 Hg to 1000 joug, followed by further doses at subsequent time intervals, necessary to maintain or to reinforce the immune response and, if required by the subject, a further dose after several months. In any case, the treatment regimen will depend on the response elicited in the treated patient, general conditions and progress of the tumor.

The pharmaceutical compositions or formulations of the present invention, both for veterinary and for human use, comprise an antigenic peptide as described above, together with one or more pharmaceutically acceptable carriers and, optionally, other therapeutic ingredients. The carrier(s) must be "acceptable" in the sense of being compatible with the other ingredients of the formulation and not deleterious to the recipient thereof. The formulations may conveniently be presented in unit dosage form and may be prepared by any method well-known in the pharmaceutical art.

All methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then, if necessary, shaping the product into the desired formulation.

Formulations suitable for intravenous, intramuscular, subcutaneous, or intraperitoneal administration conveniently comprise sterile aqueous solutions of the active ingredient with solutions which are preferably isotonic with the blood of the recipient. Such formulations may be conveniently prepared by dissolving solid active ingredient in water containing physiologically compatible substances such as sodium chloride (e.g. 0.1-2.0M), glycine, and the like, and having a buffered pH compatible with physiological conditions to produce an aqueous solution, and rendering said solution sterile. These may be present in unit or multi-dose containers, for example, sealed ampoules or vials.

The formulations of the present invention may incorporate a stabilizer. Illustrative stabilizers are polyethylene glycol, proteins, saccharides, amino acids, inorganic acids, and organic acids which may be used either on their own or as admixtures. These stabilizers are preferably incorporated in an amount of about 0.11 to about 10,000 parts by weight per part by weight of immunogen. If two or more stabilizers are to be used, their total amount is preferably within the range specified above. These stabilizers are used in aqueous solutions at the appropriate concentration and pH. The specific osmotic pressure of such aqueous solutions is generally in the range of about 0.1 to about 3.0 osmoles, preferably in the range of about 0.8 to about 1.2. The pH of the aqueous solution is adjusted to be within the range of about 5.0 to about 9.0, preferably within the range of 6-8. In formulating the immunogen of the present invention, anti-adsorption agent may be used.

The present invention also provides the use of the antigenic peptides comprising the sequences depicted in SEQ ID NOs 12 and 13, as well as the use of these peptides with deletions at the amino or carboxy terminus maintaining their binding capacity, for the manufacture of a medicament for stimulating the production of protective antibodies or immune cells with anti-tumor reactivity e.g. cytotoxic T cells or natural killer cells in a mammal.

In another embodiment, the use of a MHC class II antigenic peptide comprising an amino acid sequence selected from the group consisting of SEQ ID NOs 12 and 13 for the manufacture of a medicament for preventing or treating lung cancer by stimulating the production of protective antibodies or immune positive CD4+ T cells is provided.

Beyond that, the method of the invention can be exploited for diagnostic purposes. In one embodiment, the antigenic peptides of the invention may be used as response markers to track the efficacy of a therapeutic regime. Essentially, a baseline value for an antigenic peptide can be determined, then a given therapeutic agent is administered, and the levels of the antigenic peptide are monitored subsequently, whereas a change in the level of the antigenic peptide is indicative of the efficacy of a therapeutic treatment.

Furthermore, the antigenic peptides which are only found in certain stages or phases of a disease, preferably of cancer, may be utilized as stage-specific markers. Essentially, the levels of the antigenic peptides which have been linked to a certain disease stage are monitored regularly, thereby providing information about the stage of the disease and its progression.

Therefore, the use of a MHC class II antigenic peptide comprising an amino acid sequence selected from the group consisting of SEQ ID NOs. 12 and 13 as a diagnostic marker for cancer is provided. Preferably, the use of a MHC class II antigenic peptide of the invention as a diagnostic marker for melanoma is provided. Especially preferred is the use of a MHC class II antigenic peptide comprising an amino acid sequence selected from the group consisting of SEQ ID NOs. 12 and 13 as a diagnostic marker for melanoma.

Additionally, the use of a MHC class II antigenic peptide comprising an amino acid sequence selected from the group consisting of SEQ ID NOs. 12 and 13 as a diagnostic marker for lung cancer is provided.

The invention also includes the use of the melanotransferrin polypeptide the antigenic peptides are derived from as a marker for the diagnosis and monitoring of lung cancer. The rationale for the use of the respective proteins is that DCs reside in most tissues where they capture exogenous antigens via specific receptors and via specialized endocytotic mechanisms (e.g. macropinocytosis) followed by presentation of the processed antigens as peptides on MHC class II molecules. Previous studies have shown that the frequency of a peptide epitope found in the context of MHC class II molecules, in the majority of cases mirrors the abundance of the protein from which this particular peptide was derived from. Therefore, not only the antigenic peptides but also the corresponding proteins can serve as markers for lung cancer.

The diagnosis of a disease, preferably of cancer can be made by examining expression and/or composition of a polypeptide or peptide marker for a disease, preferably for cancer, by a variety of methods, including enzyme linked immunosorbent assays (ELISAs), Western blots, immunoprecipitations and immunofluorescence. A test sample from an individual is assessed for the presence of an alteration in the expression and/or an alteration in composition of a polypeptide or a peptide of the present invention. An alteration in expression of a polypeptide or peptide can be, for example, an alteration in the quantitative polypeptide expression (i.e., the amount of polypeptide produced); an alteration in the composition of a polypeptide is an alteration in the qualitative polypeptide expression (e.g., expression of a mutant polypeptide or of a different splicing variant).

Both such alterations (quantitative and qualitative) can also be present. An "alteration" in the polypeptide expression or composition, as used herein, refers to an alteration in expression or composition in a test sample, as compared with the expression or composition of the peptide or polypeptide in a control sample. A control sample is a sample that corresponds to the test sample (e.g., is from the same type of cells), and is from an individual who is not affected by a disease, preferably by cancer. An alteration in the expression or composition of the peptide or polypeptide in the test sample, as compared with the control sample, is indicative of a disease, preferably of cancer, or a susceptibility to a disease, preferably to cancer. Various means of examining expression or composition of a peptide or polypeptide of the present invention can be used, including spectroscopy, colorimetry, electrophoresis, isoelectric focusing, and immunoassays (*e*.*g*., David et al., U.S. Pat. No. 4,376,110) such as immunoblotting (see also Current Protocols in Molecular Biology, particularly chapter 10). For example, in one embodiment, an antibody capable of binding to the polypeptide (e.g., as described above), preferably an antibody with a detectable label, can be used. Antibodies can be polyclonal, or more preferably, monoclonal. An intact antibody, or a fragment thereof (*e.g.,* Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.

Western blotting analysis, using an antibody as described above that specifically binds to a peptide or polypeptide of the present invention, may be used to measure the level or amount of a peptide or polypeptide in a test sample and comparing it with the level or amount of the peptide or polypeptide in a control sample. Preferably the peptide or polypeptide in a test sample is measured in a homogenous or a heterogeneous immuno assay. A level or amount of the polypeptide in the test sample that is higher or lower than the level or amount of the polypeptide in the control sample, such that the difference is statistically significant, is indicative of an alteration in the expression of the polypeptide, and is diagnostic for a disease, preferably for cancer or a susceptibility to a disease, preferably to cancer.

Therefore, the present invention also relates to a diagnostic composition comprising an antibody reactive with a MHC class II antigenic peptide of the invention.

Having now generally described this invention, the same will become better understood by reference to the specific examples, which are included herein for purpose of illustration only and are not intended to be limiting unless otherwise specified, in connection with the following figures.

### Examples

The examples below are in connection with the figures described above and based on the technology illustrated in Figure 1 and described in detail in the following. Commercially available reagents referred to in the examples were used according to manufacturer's instructions unless otherwise indicated.

### Methodology of the invention

### Cell lines and culture

The study was performed with human dendritic cells which were differentiated from monocytes, as described below. Monocytes were purified from human peripheral blood. In addition, the melanoma cell lines UKRV-Mel-15a, UKRV-Mel-20c and Ma-Mel-18a (Eichmueller S et al., Exp Dermatol 2002; 11, 292-31) were utilized.

All cells were cultured in RPMI 1640 medium (short: RPMI) supplemented with 1 mM Pyruvat, 2 mM Glutamine and 10% heat-inactivated fetal calf serum (Gibco BRL, Rockville, MD).

### Isolation of peripheral blood mononuclear cells (PBMCs)

Peripheral blood was obtained from the local blood bank as standard buffy coat preparations from healthy donors. Heparin (200 I.U./ml blood, Liquemine, Roche) was used to prevent clotting. Peripheral blood mononuclear cells (PBMCs) were isolated by centrifugation in LSM^{®} (1.077-1.080g/ml; ICN, Aurora, OH) at 800g (room temperature) for 30 min. PBMCs were collected from the interphase and washed twice in RPMI containing 20 mM Hepes (500g for 15 min, 300g for 5 min). In order to remove erythrocytes, PBMCs were treated with ALT buffer (140 mM ammonium chloride, 20 mM Tris, pH 7.2) for 3 min at 37°C. PBMCs were washed twice with RPMI containing 20 mM Hepes (200g for 5 min).

### Generation of dendritic cells from peripheral blood monocytes.

Monocytes were isolated from PBMCs by positive sorting using anti-CD14 magnetic beads (Miltenyi Biotech, Auburn, CA) according to the manufacturer's protocol. Monocytes were cultured in RPMI supplemented with 1% non-essential amino acids (Gibco, BRL, Rockville, MD), 50 ng/ml recombinant human granulocyte macrophage-colony stimulating factor (GM-CSF; S.A. 1.1x10⁷U/mg) (Leucomax; Novartis, Basel Switzerland) and3 ng/ml recombinant human IL-4 (S.A. 2.9x10⁴U/µg) (R&D Systems, Minneapolis, MN). Monocytes were seeded at 0.3 x 10⁶/ml in 6-well plates (Costar) for 5 days to obtain immature dendritic cells.

The quality of monocyte-derived immature dendritic cells was routinely monitored by flow-cytometric analysis conforming to the phenotype: CD1a (high), CD3 (neg.), CD14 (low), CD19 (neg.), CD56 (neg.), CD80 (low), CD83 (neg.), CD86 (low) and HLA-DR (high). In contrast, mature dendritic cells (cf. below) display the following phenotype: CD1a (low), CD80 (high), CD83 (high), CD86 (high) and HLA-DR (high). Monoclonal antibodies against CD1a, CD3, CD14, CD19, CD56, CD80, CD83, CD86 as well as the respective isotype controls were purchased from Pharmingen (San Diego, CA).

### Exposure of dendritic cells to necrotic melanoma cells

Melanoma cells lines were rendered necrotic by 4 cycles of freezing in liquid nitrogen and subsequent thawing at room temperature. The percentage of necrotic cells was monitored by light microscopy. To feed dendritic cells with melanoma cell-derived antigen, 6 x 10⁶ immature dendritic cells were exposed to 1.8 x 10⁷ necrotic cells (3:1 ratio). At the same time maturation of dendritic cells was induced by adding 10 ng/ml recombinant human tumor necrosis factor (TNFα; S.A. 1.1x10⁵U/µg). As a control, 6 x 10⁶ dendritic cells were incubated with TNFα alone.

After 24-48 hrs of co-culture, mature dendritic cells were harvested by centrifugation at 300g for 10 min. Cells were washed with RPMI containing 10% FCS and transferred to an eppendorf tube. After centrifugation at 400g for 3 min, the supernatant was completely removed and the cells were frozen at -70°C.

### Generation of anti-HLA class II beads

The anti-HLA-DR monoclonal antibody (mAb) L243 (ATCC, Manassas, VA) was produced by culturing the respective mouse hybridoma cell line. mAb L243 was purified using ProteinA sepharose (Pharmacia, Uppsala, Sweden) and immobilized to CNBr-activated sepharose beads (Pharmacia) at a final concentration of 2.5 mg/ml, according to the manufacturer's protocol. L243 beads were stored in PBS containing 0.1% Zwittergent 3-12 (Calbiochem, La Jolla, CA).

### Nano-scale purification of HLA-DR-peptide complexes

Pellets of frozen dendritic cells were resuspended in 10-fold volume of ice cold lysis buffer (1% Triton-X-100, 20 mM Tris, pH 7.8, 5 mM MgCl₂, containing protease inhibitors chymostatin, pepstatin, PMSF and leupeptin (Roche, Mannheim, Germany)) and lysed in a horizontal shaker at 1000 rpm, 4°C for 1h. The cell lysate was cleared from cell debris and nuclei by centrifugation at 2000g, 4°C for 10 min. The lysate was co-incubated with L243 beads (5-10 µl L243 beads per 100 µl cell lysate) in a horizontal shaker at 1000 rpm, 4°C for 2 hrs. Immunoprecipitated HLA-DR-peptide complexes bound to L243 beads were sedimented by centrifugation at 2000g, 4°C for 5 min and washed three times with 300 µl 0.1% Zwittergent 3-12 (Calbiochem) in PBS.

The efficacy of depletion of HLA-DR-peptide complexes was monitored by analyzing the respective cell lysates before and after immunoprecipitation. In parallel, aliquots of the beads were analyzed by western blotting using the anti-HLA-DRα-specific mAb 1B5 (Adams, T.E. et al., Immunology 50 (1983) 613-624).

### Elution of HLA-DR-associated peptides

HLA-DR-peptide complexes bound to L243 beads were resuspended in 400 µl H₂O (HPLC-grade; Merck, Darmstadt, Germany), transferred to an ultrafiltration tube, Ultrafree MC, 30 kD cut-off (Millipore, Bedford, MA) and washed 10 times with 400 µl H₂O (HPLC-grade) by centrifugation for 2-4 min at 14000 rpm at 4°C. For eluting the bound peptides, 50 µl 0.1% trifluoracetic acid (Fluka, Buchs, Switzerland) in H₂O (HPLC-grade) was added and incubation was performed for 30 min at 37°C. Eluted peptides were collected in a new eppendorf tube by centrigugation of the Ultrafree unit at 14000 rpm for 3 min at RT and immediately lyophilized in a Speed-Vac^{™} vacuum centrifuge.

### Fractionation of peptides by nano-HPLC

Lyophilized peptides eluted from HLA-DR molecules were resolved in 0.05% trifluoroacetic acid, 5% acetonitrile (Merck, Darmstadt, Germany) in H2O, (HPLC-grade) and separated on a 75 µm x 15 cm C18 PepMap capillary (C18; 3µm; 100 Å) (LC-Packings, Amsterdam, Netherlands) connected to a FAMOS^{™} autosampler and an ULTIMATE^{™} nano-flow HPLC (Dionex, Olten, Switzerland). The following non-linear gradient at a constant flow rate of 200 nl/min was used: 0-40min 5-50% system B; 40-50 min 50-90% system B. System A was 0.05% trifluoroacetic, 5% acetonitrile/H2O and system B was 0.04% trifluoroacetic, 80% acetonitrile/H2O. The separation was monitored via dual UV absorption at 214 nm and 280 nm. Fractions (400 nl) were collected using the fraction collector PROBOT^{™} (BAI, Weiterstadt, Germany) and spotted onto an AnchorChip 600/384 MALDI-MS target (Bruker, Bremen, Germany).

### Sequence analysis of peptides by mass spectrometry

### MALDI-TOF mass spectrometry

Peptides spotted onto an AnchorChip plate were co-cristallized with matrix (10 mg/ml; α-cyano-4-hydroxy-cinnamic acid (Merck, Darmstadt, Germany), 50% acetonitrile, 0.1% trifluoroacetic acid). For qualitative analysis of the whole peptide repertoire, samples were analyzed on an Ultraflex^{™} MALDI-TOF mass spectrometer (Bruker, Bremen, Germany), according to the manufacturer's protocol.

### Ion Trap MS/MS mass spectrometry

To perform high-throughput sequencing of complex peptide mixtures, the MudPIT (multidimensional protein identification technology) was used (Washburn MP et al., Nat Biotechnol 19 (2001), 242-247) which is based on a liquid chromatographic fractionation followed by mass spectrometric sequencing.

To this end, the lyophilized peptides eluted from HLA molecules were resuspended in a buffer containing 5% (v/v) acetonitrile, 0.5% (v/v) acetic acid, 0.012% (v/v) heptafluoro butyric acid (HFBA) and 5% (v/v) formic acid. The sample was separated on a fused-silica microcapillary column (100 µm i.d. × 365 µm) generated by a Model P-2000 laser puller (Sutter Instrument Co., Novato, CA). The microcolumn was packed with 3 µm / C18 reverse-phase material (C18-ACE 3 µm [ProntoSIL 120-3-C18 ACE-EPS, Leonberg, Germany]) followed by 3 cm of 5 µm cation exchange material (Partisphere SCX;Whatman, Clifton, NJ).

A fully automated 8-step gradient separation on an Agilent 1100 series HPLC (Agilent Technologies, Waldbronn, Germany) was carried out, using the following buffers: 5% ACN/0.02% HFBA/0.5% acetic acid (buffer A), 80% ACN/0.02% HFBA/0.5% acetic acid (buffer B), 250 mM ammonium acetate/5% ACN/0.02% HFBA/0.5% acetic acid (buffer C), and 1.5 M ammonium acetate/5% ACN/0.02% HFBA/0.5% acetic acid (buffer D). The first step of 106 min consisted of a 100 min gradient from 0 to 80% buffer B and a 6 min hold at 80% buffer B. The next 6 steps (106 min each) are characterized by the following profile: 5 min of 100% buffer A, 2 min of x% buffer C, 5 min of 100% buffer A, a 3 min gradient from 0 to 10% buffer B, a 55 min gradient from 10 to 35% buffer B, a 20 min gradient from 35 to 50% buffer B, a 16 min gradient from 50 to 80% buffer B. The 2 min buffer C percentages (x) in steps 2-7 were as follows: 10, 20, 30, 40, 70, 90, and 100%. Step 8 consisted of the following profile: a 5 min 100% buffer A wash, a 20 min salt wash with 100% buffer D and a 100 min gradient from 0-80% buffer B.

The HPLC column was directly coupled to a Finnigan LCQ ion trap mass spectrometer (Finnigan, Bremen, Germany) equipped with a nano-LC electrospray ionization source. Mass spectrometry in the MS-MS mode was performed according to the manufacturer's protocol. The identification of peptides was done by the sequest algorithm against the swiss.fasta database.

### MALDI-PSD mass spectrometry

As an alternative to doing sequence analysis by ion trap MS/MS, as described above, MALDI-PSD analysis was performed on a Bruker Ultraflex TOF/TOF mass spectrometer (Bruker, Bremen, Germany) using the software FLEXControl 1.1 Alpha for data acquisition. Calibration was achieved by using a tryptic digest of human serum albumin (Merck, Darmstadt, Germany). Peptide mixtures were first scanned in a reflectron mode. Peptides of interest were then selected for lift mode (MALDI-PSD analysis). The peptide fragmentation spectra obtained were automatically evaluated using the Xmas 5.1.2 and Biotools 2.1 Software (Bruker) and used for sequence identification in a non-redundant protein database using the MASCOT algorithm (http://www.matrixscience.com).

### Peptide binding assay

### Peptide synthesis

Peptides were synthesized using F-moc chemistry and were purified by reverse-phase high performance liquid chromatography (RP-HPLC). Some peptides were biotinylated by coupling biotinyl-amino-hexanoic acid at the N-terminus during F-moc synthesis. Purity of peptides was routinely checked by MALDI-MS.

### Purification of HLA-DR molecules

HLA-DR molecules were purified from 10¹⁰ EBV-transformed B cell lines or T2-transfectants by affinity chromatography using anti-DR mAb L243, as described (Kropshofer H., et al., Proc. Natl. Acad. Sci. USA. 1995; 92, 8313-8317).

### In vitro peptide binding assay

HA(307-319), PKYVKQNTLKLAT, is an immunodominant epitope from influenza virus hemagglutinin that binds well to HLA-DR1, DR4 and DR5 and was used as the reporter peptide.

Purified detergent-solubilized HLA-DR1, HLA-DR4 or HLA-DR5 molecules (200 nM) were co-incubated with biotinylated HA(307-319) peptide (200 nM) and graded amounts of competitor peptide (100 nM - 10 µM) for 24 hrs at 37°C in binding buffer (50 mM sodium phosphate, 50 mM sodium citrate, pH 5.0, 0.1 % Zwittergent 3-12) in a total volume of 50 µl.

3 x 10 µl were diluted 10-fold in PBS containing 0.05% Tween-20 and 1 % BSA and incubated in a microtiterplate (Nunc), coated with the anti-DR mAb L243, for 3 hours. Plates were developed by incubation for 45 min with 0.1 µg/ml streptavidin-europium (Wallay Oy) according to the manufacturer's protocol. Quantification of binding of biotinylated HA(307-319) peptide to HLA-DR molecules was performed utilizing time-resolved Europium fluorescence and the VICTOR multilabel counter (Wallac Oy) (Arndt SO et al., EMBO J. 2000; 19, 1241-1251).

### T cell recognition

### Isolation of T cells

CD4+ T cells were isolated from PBMCs by negative selection using the CD4+ T cell isolation kit (Milteny Biotech) consisting of a hapten antibody cocktail and anti-hapten antibodies coupled to magnetic beads. T cells were cultured in RPMI medium supplemented with 1% autologous human serum, 1% non-essential amino acids (Gibco, BRL), 1% sodium pyruvate (Gibco, BRL), 1% Kanamycine (Gibco, BRL) and 1% Glutamate (Gibco, BRL). Quality control of isolated CD4+ T cells was performed by flow-cytometric analysis to show the following phenotype: CD3 (high), TcR (high), CD4 (high), CD8 (neg), CD19 (neg), CD45RO/RA (high).

### Generation of a tumor antigen specific T cell line

1x10⁶ T cells were initially stimulated with 2x10⁵ autologous dendritic cells that were pulsed with lipopolysaccharide (LPS from Salmonella abortus equi, Sigma) and 20 µM melanotransferrin peptide. After 5 days IL-2 (1250U/ml) was added. Every 10-14 days the responding T cells were restimulated with autologous dendritic cells pulsed with 20 µM melanotransferrin peptide (SEQ ID NO: 13) and grown in medium containing IL-2. After every round of restimulation the specificity of the growing T cells was assessed by sandwich immunoassays for IFN-γ and IL-4.

### STEP analysis

Single target expression profiling (STEP) was performed on a plate containing cancer and normal tissues from a variety of sources (testis, brain, spleen, muscle, lymph, adipose, lung, lung cancer, melanoma, colon, colon cancer, colon cancer metastasis, prostate, prostate cancer): Total RNA was extracted from snap-frozen human tissues using 'Ultraspec RNA isolation kits' (Biotecx, BL10100), and further purified using RNeasy mini kits (Qiagen). Fifteen µg total RNA was converted into double-stranded cDNA by reverse transcription (GIBCO BRL Life Technologies, Grand Island, NY) using the T7-T24 primer (5'-GGC CAG TGA ATT GTA ATA CGA CTC ACT ATA GGG AGG CGG (dT₂₄)) and cleaned up by Phenol/Chloroform/Isoamyl extraction using phase lock gel (5 Prime-3 Prime Inc.). Master 384-well plates were generated containing 5 ng/µl double-stranded cDNA derived from total RNA using known methods. Daughter plates were produced (final cDNA concentration: 40 pg/µl (200 pg/well)) either manually or via robotics. Duplex Real-Time PCR (target gene and GAPDH as reference gene) on 384-well optical plates was performed using TaqMan^{®} technology and analyzed on an ABI Prism^{®} PE7900 Sequence Detection System (Perkin-Elmer Applied Biosystems (PE), Lincoln, CA), which uses the 5'nuclease activity ofTaq DNA polymerase to generate a real-time quantitative DNA analysis assay. PCR mix per well (25 µl) consisted of commercially available, premixed GAPDH TaqMan^{®} primers/probe (PE), 900nM each of 5' and 3' primers and 200nM TaqMan^{®} probe from each target gene, 200pg cDNA and TaqMan^{®} Universal PCR Master Mix (PE). The following PCR conditions were used: 50°C for 2 minutes, then 95°C for 10 minutes, followed by 40 cycles at 95°C for 15 seconds and 62°C for 1 minute.

The following primer pair was generated, specifically picking up a sequence from exon 9 of the melanotransferrin gene therefore being restricted to the long transcript of melanotransferrin from which the antigenic epitope was derived:
5'-Mtf: CAGTGCGTGTCAGCCAAGTC (SEQ ID NO: 14);
3'-Mtf: TTCCCCGCCGTGTAAATGT (SEQ ID NO: 15)

The following site specific probe sequence labeled with a fluorescent reporter dye and a fluorescent quencher dye was used for detection:
P-Mtf: AGCGTCGACCTGCTCAGCCTGG (SEQ ID NO: 16)

The relative expression of the gene of interest was calculated with the equation 2^{ΔcT}. ΔcT is the difference in the thermocycles of the GAPDH gene vs. gene of interest after which the fluorescent signal pierces the threshold. The expression of GAPDH in each tissue was adjusted to the expression level of a panel of 8 housekeeping genes.

### Antibodies

The mouse monoclonal antibody L235 is a melanotransferrin specific antibody of the IgG1 isotype. The hybridoma cell line producing this antibody was purchased from ATCC (HB-8446).

### Results

The identified epitope of melanotransferrin can be recognized by T-cells (Fig. 4). Repeated restimulation of T cells from a HLA-DR4 positive donor with autologous dendritic cells that were pulsed with the identified melanotransferrin epitope and LPS resulted in the generation of a T-cell line specifically recognizing the epitope. The induced T-cell line exclusively secretes IFN-γ but not IL-4. The T-cell response is titratable depending on the dose of antigen. Thus the identified melanotransferrin epitope is immunogenic and therefore a candidate for a peptide vaccine.

Furthermore, the identified epitope could also be eluted from HLA-DR molecules of the melanoma cell line UKRV-Mel-17 that strongly expresses the melanotransferrin protein and is HLA-DR4 positive. Furthermore, a novel MART-1 antigenic peptide (SEQ ID NO: 21) has been eluted and identified from HLA-DR molecules of the same cell line (Table 4). MART-1 is already known as a tumor antigen (Kawakami Y., Eliyahu S., Delgado C.H., Robbins P.F., Rivoltini L., Topalian S.L., Miki T., Rosenberg S.A., Proc. Natl. Acad. Sci. U.S.A. 91:3515-3519(1994); Coulie P.G., Brichard V., van Pel A., Woelfel T., Schneider J., Traversari C., Mattei S., de Plaen E., Lurquin C., Szikora J.-P., Renauld J.-C., Boon T., J. Exp. Med. 180:35-42(1994)).

The melanotransferrin protein is expressed on most melanoma cell lines that were assessed with some showing very strong expression (Fig. 5). Dendritic cells that were used to identify the novel antigen taken up from necrotic Ma-Mel 18a cells do not express melanotransferrin by themselves.

mRNA expression profiling using a panel of normal vs. cancer tissues revealed that melanotransferrin is largely absent on all normal tissues that were assessed but shows strong expression on several lung cancers and to a lesser extend also colon cancer cells (Fig. 6).

With regard to the specific expression of melanotransferrin in certain tumor tissues, its broad expression in melanoma cells and the ability of the identified epitope to specifically activate T-cells the newly identified melanotransferrin epitope meet the requirements of a novel tumor antigen that may be used for peptide vaccinations.

### Example 1

The above described methodology (Fig. 1) was used to identify novel HLA-DR-associated tumor peptides derived from (or induced by) the melanoma cell line, UKRV-Mel-15a. The melanoma cell line UKRV-Mel-15a does not express HLA-DR molecules by itself.

3 x 10⁶ cells dendritic cells were co-incubated with 9 x 10⁶ necrotic cells of the melanoma line UKRV-Mel-15a and cultured for 24 hrs in the presence of TNFα, (10 ng/ml). As a control, 3 x 10⁶ cells dendritic cells were cultured in the presence of TNFα (10 ng/ml) only.

Both sets of dendritic cells were lysed in detergent TX-100 and HLA-DR molecules were precipitated using anti-DR mAb L243. HLA-DR associated peptides were eluted with 0.1% TFA and analyzed by MALDI-MS (Fig. 2A):
In this example, the HLA-DR associated peptides from both DC cultures were compared by MALDI-MS spectrometry and only the peptide signals contained in the profile of DCs pulsed with melanoma cells were used to identify new epitopes by successive sequencing.

MALDI-MS analysis revealed one dominant signal with an observed mass of m/z = 1820.6 in the spectrum of pulsed DCs as compared to unpulsed DCs (Fig. 2A).

Sequencing by MALDI-PSD fragmentation resulted in a novel epitope derived from the tumor antigen vimentin (accession number: P08670; swissprot): vimentin(202-217) with the amino acid sequence TLQSFRQDVDNASLAR (Fig. 2B; Table 1, SEQ ID NO. 1). Sequence analysis by ion trap MS-MS confirmed this sequence (Fig. 2C).

Analysis of the results of high-throughput ion trap MS/MS sequencing of the whole peptide repertoire revealed 3 further length variants of the same vimentin epitope (Table 1): the 15-mer vimentin(203-217) (SEQ ID NO. 2), the 14-mer vimentin(203-216) (SEQ ID NO. 3) and the 14-mer vimentin(202-215) (SEQ ID NO. 4). The 4 length variants of the vimentin epitope and the melanoma antigens Melan A(51-65), CDC27(768-782), tyrosinase(448-462)and gp100(44-59) share a common sequence motif suitable for binding to HLA-DR4 (DRB1*0401) (Table 2). Binding to HLA-DR4 could be confirmed in an *in vitro* binding assay involving synthetic vimentin(202-217) peptide and purified HLA-DR4 molecules (Fig. 3): according to its IC₅₀ value against the reporter peptide HA(307-319), vimentin(202-217) binds to HLA-DR4 (Fig. 3B) with high affinity, similar to CDC-27(768-782), but poorly to DR1 (Fig. 3A) or DR5 (Fig. 3C).

The vimentin(202-217) peptide identified by the method of the invention is the first vimentin derived HLA class II restricted epitope described so far.

### Example 2

The methodology was further used to identify peptides bound to HLA-DR molecules of dendritic cells (DCs) after TNFα-induced maturation and exposure to necrotic melanoma cell line UKRV-Mel-20c. The melanoma cell line UKRV-Mel-20c does not express HLA-DR molecules by itself. Sequencing was done by high-throughput ion trap MS/MS technology.

Thus, 5 x 10⁶ cells dendritic cells were co-incubated with 1.5 x 10⁷ necrotic cells of the melanoma line UKRV-Mel-20c and cultured for 24 hrs in the presence of TNFα (10 ng/ml). As a control, 5 x 10⁶ cells dendritic cells were cultured in the presence of TNFα, (10 ng/ml) only.

Both sets of dendritic cells were lysed in detergent TX-100 and HLA-DR molecules were precipitated using anti-DR mAb L243. HLA-DR associated peptides were eluted with 0.1% TFA and analyzed by LC-high-throughput ion trap MS/MS technology.

The peptide sequences identified from unpulsed DCs (control) were compared with the peptide sequences identified from DCs pulsed with necrotic melanoma cells:

35 individual peptide sequences from HLA-DR molecules of DCs were identified in the absence of melanoma cells, and 40 peptide sequences were found in the presence of UKRV-Me120c melanoma cells. Comparison of the peptide sequences revealed that 21 peptides are identical, 14 sequences (11 epitopes) are specific for unpulsed DCs and 17 sequences (9 epitopes) are only presented after melanoma cell pulse.

Importantly, 3 of the 9 melanoma cell induced epitopes are derived from known tumor marker proteins, namely translation factor eIF-4A1 (accession number: P04765; swissprot), interferon-gamma (IFNgamma)-inducible P78 (accession number: AAD43063; locus AF135187) and cytoskeletal protein vimentin (accession number: P08670; swissprot) (Table 1).

The importance of vimentin with regard to serving as a melanoma antigen is underscored by the fact that a second vimentin epitope could be identified using the melanoma cell line, UKRV-Me120c. In this case, two length variants were found: vimentin(166-183) (SEQ ID NO. 5) and vimentin(167-183) (SEQ ID NO. 6). In contrast to the former vimentin epitope, vimentin(167-183) not only carries a binding motif for HLA-DR4 (cf. Table 2), but appears to be a promiscuous HLA-DR binder, as it displays moderate to good binding in the context of HLA-DR1 (Fig. 3A), HLA-DR4 (Fig. 3B) and HLA-DR5 (Fig. 3C).

Necrotic UKRV-Mel-20c melanoma cells gave also rise to 3 peptides derived from the translation initiation factor eIF-4A1 (Table 1): one epitope is represented by 2 length variants eIF4A1(172-187) (SEQ ID NO. 7) and eIF4A1(172-186) (SEQ ID NO. 8) while the other is found as peptide eIF4A1(321-338) (SEQ ID NO. 9).

Furthermore, two peptides derived from the interferon-inducible protein p78 were identified (Table 1) being length variants of the same epitope: p78(503-516) (SEQ ID NO. 10) and p78(503-515) (SEQ ID NO. 11). The p78 protein is implicated, as yet, in prostate cancer.

Thus, the peptides derived from translation factor eIF-4A1, IFNgamma-inducible p78 and vimentin identified by the method of the invention are new candidate tumor antigens to be used as diagnostic markers or vaccines in therapeutic approaches.

### Example 3

A third melanoma cell line, Ma-Mel-18a, was utilized to identify novel HLA-DR-associated tumor peptides. The melanoma cell line Ma-Mel-18a does not express HLA-DR molecules by itself.

Thus, 4 x 10⁶ cells dendritic cells were co-incubated with 1.2 x 10⁷ necrotic cells of the melanoma line Ma-Mel-18a and cultured for 24 hrs in presence of TNFα (10 ng/ml). As a control, 4 x 10⁶ cells dendritic cells were cultured in the presence of TNFα (10 ng/ml) only.

Both sets of dendritic cells were lysed in detergent TX-100 and HLA-DR molecules were precipitated using anti-DR mAb L243. HLA-DR associated peptides were eluted with 0.1% TFA and analyzed by LC-high-throughput ion trap MS/MS technology.

The peptide sequences identified from the control (unpulsed DCs) were compared with the peptide sequences identified from DCs pulsed with necrotic Ma-Mel-18a (Table 3): In the absence of Ma-Mel-18a cells 155 individual self-peptide sequences derived from 75 self-proteins were found. 22 of these self-peptides vanished upon encounter of necrotic Ma-Mel-18a cells; however 26 of 165 self-peptides have not been found in the control. These 26 self-peptides, which were obviously induced by Ma-Mel-18a melanoma cells, were derived from 19 proteins. 18 of the 19 proteins were either expressed by DCs only or by both melanoma cells and DCs. None of the 18 proteins have been described in the context of melanoma or other tumors.

The only protein not expressed by DCs but by Ma-Mel-18a cells was melanotransferrin (accession number: P08582; swissprot): Two length variants of the same p97 epitope were identified (Table 1): p97(688-684) (SEQ ID NO. 12) and p97(688-683) (SEQ ID NO. 13).

p97 is a well known melanoma marker protein, however, p97-derived tumor antigenic peptides have not been defined, as yet.

Similar to both vimentin epitopes described above (Table 1, 2), the p97 epitope contains the peptide binding motif of HLA-DR4 with L-672, D-675, T-677 and A-680 serving as P1, P4, P6 and P9 anchor, respectively (Table 2). In agreement with that, p97(668-683) binds with high affinity to HLA-DR4 (Fig. 3B). Moreover, it binds with moderate affinity to HLA-DR1 (Fig. 3A) and HLA-DR5 (Fig. 3C).

In further accordance with these results, DCs which led to the identification of the p97 results, DCs which led to the identification of the p97 peptides were expressing HLA-DR4 and HLA-DR1. Therefore, the p97 epitope described here may be utilized in the context of a variety of HLA-DR alleles.

With regard to the broad expression of melanotransferrin in melanoma tissues (Palmieri G et al., J. Clin. Oncol. 1999; 17, 304-311) the melanotransferrin epitope may serve as a candidate tumor antigen.

Thus, peptide vaccines based on melanotransferrin leading to MHC class II- and CD4+ T cell mediated immune responses, should be most suitable in therapy against melanotransferrin-expressing tumors.

**TABLE 1**

| **HLA-DR associated peptide antigens induced by melanoma cells** | | | | | |
|---|---|---|---|---|---|
| SEQ. ID No. | LENGTH | MELANOMA CELL ^{a} | SEQUENCE^{b} | POSTITION^{c} | PROTEIN SOURCE |
| 1 | 16 | UKRV-Mel-15a | TLQSFRQDVDNASLAR | 202-217 | Vimentin |
| 2 | 15 | UKRV-Mel-15a | LQSFRQDVDNASLAR | 203-217 | Vimentin |
| 3 | 14 | UKRV-Mel-15a | LQSFRQDVDNASLA | 203-216 | Vimentin |
| 4 | 14 | UKRV-Mel-15a | TLQSFRQDVDNASL | 202-215 | Vimentin |
| 5 | 18 | UKRV-Mel-20C | NDKARVEVERDNLAEDIM | 166-183 | Vimentin |
| 6 | 17 | UKRV-Mel-20c | DKARVEVERDNLAEDIM | 167-183 | Vimentin |
| 7 | 16 | UKRV-Met-20c | SPKYIKMFVLDEADEM | 172-187 | eIF-4A1 |
| 8 | 15 | UKRV-Mel-20c | SPKYIKMFVLDEADE | 172-186 | eIF-4A1 |
| 9 | 18 | UKRV-Mel-20c | GSSRVLITTDLLARGIDV | 321-338 | eIF-4A1 |
| 10 | 14 | UKRV-Mel-20c | KSKIEDIRAEQERE | 503-516 | IFN-induc. p78 |
| 11 | 13 | UKRV-Mel-20c | KSKIEDIRAEQER | 503-515 | IFN-induc. p78 |
| 12 | 17 | Ma-Mel-18a Melanotransferrin | GQDLLFKDATVRAVPVG | 668-684 | |
| 13 | 16 | Ma-Mel-18a Melanotransferrin | GQDLLFKDATVRAVPV | 668-683 | |

| | | | | | |
|---|---|---|---|---|---|
| ^{a} Name of the melanoma cell line used after necrotization for pulsing dendritic cells. ^{b} Sequences of the melanoma cell-derived peptides in one-letter-code ^{c} Position of the epitope within the protein sequence | | | | | |

**TABLE 2**

| **Melanoma cell-derived peptide antigens sharing the binding motif of HLA-DR4 (DRB1*0401)** | | | | | |
|---|---|---|---|---|---|
| SEQ. ID No. | LENGTH | SEQUENCE^{a} | POSTITION^{b} | PROTEIN SOURCE | REFERENCE |
| 1 | 16 | TLQSFRQDVDNASLAR | 202-217 | Vimentin | this study |
| 2 | 15 | LQSFRQDVDNASLAR | 203-217 | Vimentin | this study |
| 3 | 14 | LQSFRQDVDNASLA | 203-216 | Vimentin | this study |
| 4 | 14 | TLQSFRQDVDNASL | 202-215 | Vimentin | this study |
| 5 | 18 | NDKARVEVERDNLAEDIM | 166-183 | Vimentin | this study |
| 6 | 17 | DKARVEVERDNLAEDIM | 167-183 | Vimentin | this study |
| 12 | 17 | GQDLLFKDATVRAVPVG | 668-684 | Melanotransferrin | this study |
| 13 | 16 | GQDLLFKDATVRAVPV | 668-683 | Melanotransferrin | this study |
| 14 | 15 | RNGYRALMDKSLHVG | 51-65 | Melan-A | b,c |
| 15 | 15 | MNFSWAMDLDFKGAN | 768-782 | CDC-27 | b,c |
| 16 | 15 | DYSYLQDSDPDSFQD | 448-462 | Tyrosinase | b,c |
| 17 | 16 | WNRQLYPEWTEAQRLD | 44-59 | gp100 | c |

| | | | | | |
|---|---|---|---|---|---|
| ^{a} The sequences of the peptides are given in one-letter-code and are aligned according to the peptide binding motif of HLA-DR4 (DRB1*0401): P1 anchor: W,Y,F,I,L,V; P4 anchor: D,E; P6 anchor: T,S,A; P9 anchor: A,S,G. The core epitope region encompassing P1-P9 is underlined. ^{b} R.-F. Wang, Trends in Immunology 22, 269-276 (2001). ^{c} N. Renkvist et al., Cancer Immunol. Immunother. 50, 3-15 (2001).- | | | | | |

**TABLE 3**

| **The HLA-DR associated peptide repertoire of DCs (DRB1*0101/DRB1*0401) in the absence and presence of necrotic Ma-Mel-18a** | | | | | |
|---|---|---|---|---|---|
| | peptides | proteins^{a} | maximal number of peptides / protein | unique peptides^{b} | unique proteins^{b} |
| TNF | 155 | 75 | 8 | 22 | 20 |
| TNF + melanoma cells | 165 | 75 | 10 | 26 | 19 |

| | | | | | |
|---|---|---|---|---|---|
| ^{a} number of proteins giving rise to the identified peptides ^{b} number of peptides or proteins which were found exclusively in the absence or exclusively in the presence of Ma-Mel-18a cells. | | | | | |

**TABLE 4**

| **MHC class II restricted epitopes of melanoma associated proteins eluted from MHC class II molecules of HLA-DR4 positive melanoma cell line UKRV-Mel-17** | | | |
|---|---|---|---|
| SEQ. ID NO. | LENGTH | SEQUENCE | PROTEIN SOURCE |
| 21 | 17 | APPAYEKLSAEQSPPPY | melanoma antigen recognized by T-cells 1 (MART-1) (Melan-A protein) |
| 12 | 17 | GQDLLFKDATVRAVPVG | Melanotransferrin (p97 antigen) |

## Claims

1. An isolated MHC class II antigenic peptide comprising an amino acid sequence selected from the group consisting of SEQ ID NOs. 12 to 13.

2. The antigenic peptide of claim 1, wherein the peptide has amino acid deletions at the carboxy or amino terminus and maintains binding capacity.

3. The antigenic peptide of any one of claims 1 to 2 linked to a MHC class II molecule.

4. Antibodies reactive with an antigenic peptide of claim 1 to 2.

5. An isolated nucleic acid molecule encoding a peptide or polypeptide according to any one of claims 1 to 3.

6. A recombinant nucleic acid construct comprising the nucleic acid molecule of claim 5 operably linked to an expression vector.

7. A host cell containing the nucleic acid construct according to claim 6.

8. A method for producing a MHC class II antigenic peptide according to any one of claims 1 to 3 comprising the steps of culturing the host cell of claim 7 under conditions allowing expression of said peptide and recovering the peptide from the cells or the culture medium.

9. A pharmaceutical composition comprising the antigenic peptide of any one of claims 1 to 3 and an acceptable excipient, diluent or carrier.

10. The isolated antigenic peptide of any one of claims 1 to 3 for use in the diagnosis, prevention and treatment of a disease, preferably of cancer.

11. The use of a MHC class II antigenic peptide according to any one of claims 1 to 2 as a diagnostic marker for cancer.

12. The use according to claim 11, wherein the MHC class II antigenic peptide is a diagnostic marker for melanoma.

13. The use of a MHC class II antigenic peptide according to claim 1 as a diagnostic marker for lung cancer.

14. The use of the antigenic peptides of any one of claims 1 to 3 for the manufacture of a medicament for stimulating the production of protective antibodies or immune cells in a mammal.

15. The use of the antigenic peptides of any one of claims 1 to 3 for the manufacture of a medicament for preventing or treating melanoma by stimulating the production of protective antibodies or immune positive CD4+ T cells.

16. The use of a MHC class II antigenic peptide according to claim 1 for the manufacture of a medicament for preventing or treating lung cancer by stimulating the production of protective antibodies or immune positive CD4+ T cells.

## Revendications

1. Peptide antigène MHC de classe II comprenant une séquence d'acides aminés choisis dans le groupe consistant en SEQ ID n° 12 à 13.

2. Peptide antigène selon la revendication 1, dans lequel le peptide présente des délétions d'acides aminés au niveau de la terminaison carboxy ou amino et conserve une capacité de liaison.

3. Peptide antigène selon l'une quelconque des revendications 1 à 2, lié à une molécule MHC de classe II.

4. Anticorps capable de réagir avec un peptide antigène selon la revendication 1 ou 2.

5. Molécule d'acide nucléique isolée codant un peptide ou polypeptide selon l'une quelconque des revendications 1 à 3.

6. Construction d'acide nucléique recombinant comprenant la molécule d'acide nucléique de la revendication 5 liée de façon opérante à un vecteur d'expression.

7. Cellule hôte contenant la construction d'acide nucléique selon le revendication 6.

8. Procédé pour produire un peptide antigène MHC de classe II selon l'une quelconque des revendications 1 à 3, comprenant les étapes consistant à mettre en culture la cellule hôte de la revendication 7 dans des conditions permettant l'expression dudit peptide et à récupérer le peptide à partir des cellules ou du milieu de culture.

9. Composition pharmaceutique comprenant le peptide antigène selon l'une quelconque des revendications 1 à 3 et un excipient acceptable, diluant ou véhicule.

10. Peptide antigène isolé selon l'une quelconque des revendications 1 à 3 pour l'utilisation dans le diagnostic, la prévention et le traitement d'une maladie, de préférence un cancer.

11. Utilisation d'un peptide antigène MHC de classe II selon l'une quelconque des revendications 1 à 2 en tant que marqueur de diagnostic pour le cancer.

12. Utilisation selon la revendication 11, dans laquelle le peptide antigène MHC de classe II est un marquer de diagnostic pour les mélanomes.

13. Peptide antigène MHC de classe II selon la revendication 1 en tant que marqueur de diagnostic pour le cancer du poumon.

14. Utilisation des peptides antigènes selon l'une quelconque des revendications 1 à 3 pour la fabrication d'un médicament destiné à stimuler la production d'anticorps protecteurs ou de cellules immunes chez un mammifère.

15. Utilisation des peptides antigènes selon l'une quelconque des revendications 1 à 3 pour la fabrication d'un médicament destiné à la prevention ou au traitement des mélanomes par stimulation de la production des anticorps protecteurs ou des cellules positives immunes CD4+T.

16. Utilisation d'un peptide antigène MHC de classe II selon la revendication 1 pour la fabrication d'un médicament destiné à la prevention ou au traitement du cancer du poumon par stimulation de la production des anticorps protecteurs ou des cellules immunes positives CD4+T.

## Patentansprüche

1. Isoliertes MHC-Klasse-II-Antigenpeptid, umfassend eine Aminosäuresequenz, ausgewählt aus der Gruppe, bestehend aus SEQ ID Nr. 12 bis 13.

2. Antigenpeptid nach Anspruch 1, wobei das Peptid Aminosänredeletionen am Carboxy- oder Aminoterminus aufweist und Bindungsfähigkeit aufrechterhält.

3. Antigenpeptid nach einem der Ansprüche 1 bis 2, das an ein MHC-Klasse-II-Molekül gebunden ist.

4. Antikörper, die mit einem Antigenpeptid nach Anspruch 1 bis 2 reagieren können.

5. Isoliertes Nukleinsäuremolekül, das ein Peptid oder Polypeptid nach einem der Ansprüche 1 bis 3, kodiert.

6. Rekombinantes Nukleinsäurekonstrukt, umfassend das operabel an einen Expressionsvektor gebundene Nukleinsäuremolekül nach Anspruch 5,

7. Wirtszelle, enthaltend das Nukleinsäurekonstrukt nach Anspruch 6.

8. Verfahren zur Herstellung eines MHC-Klasse-II-Antigenpeptids nach einem der Ansprüche 1 bis 3, umfassend die Schritte der Kultivierung der Wirtszelle nach Anspruch 7 unter Bedingungen, die die Expression des Peptids ermöglichen, und der Gewinnung Peptids aus den Zellen oder dem Kulturmedium.

9. Pharmazeutische Zusammensetzung, umfassend das Antigenpeptid nach einem der Ansprüche 1 bis 3 und einen akzeptablen Hilfsstoff, ein akzeptables Verdünnungsmittel oder einen akzeptablen Träger.

10. Isoliertes Antigenpeptid nach einem der Ansprüche 1 bis 3 zur Verwendung bei der Diagnose, Vorbeugung und Behandlung einer Krankheit, bevorzugt von Krebs.

11. Verwendung eines MHC-Klasse-II-Antigenpeptids nach einem der Ansprüche 1 bis 2 als diagnostischer Marker für Krebs.

12. Verwendung nach Anspruch 11, wobei das MHC-Klasse-II-Antigenpeptid ein diagnostischer Marker für ein Melanom ist.

13. Verwendung eines MHC-Klasse-II-Antigenpeptids nach Anspruch 1 als diagnostischer Marker für Lungenkrebs.

14. Verwendung der Antigenpeptide nach einem der Ansprüche 1 bis 3 zur Herstellung eines Medikaments zur Stimulierung der Produktion schützender Antikörper oder Immunzellen in einem Säuger.

15. Verwendung der Antigenpeptide nach einem der Ansprüche 1 bis 3 zur Herstellung eines Medikaments zur Vorbeugung oder Behandlung eines Melanoms durch Stimulieren der Produktion schützender Antikörper oder positiver CD4+-T-Immunzellen.

16. Verwendung eines MHC-Klasse-II-Antigenpeptids nach Anspruch 1 zur Herstellung eines Medikaments zur Vorbengung oder Behandlung von Lungenkrebs durch Stimulieren der Produktion schützender Antikörper oder positiver CD4+-T-Immunzellen.


### SEQUENCE LISTING

<110> F. Hoffmann La Roche AG
<120> Identification of novel MHC class II associated candidate tumor antigens
<130> Case 21412
<160> 22
<170> PatentIn version 3.2
<210> 1
   <211> 16
   <212> PRT
   <213> Homo sapiens
<400> 1
   Thr Leu Gln Ser Phe Arg Gln Asp Val Asp Asn Ala Ser Leu Ala Arg
   1               5                   10                  15
<210> 2
   <211> 15
   <212> PRT
   <213> Homo sapiens
<400> 2
   Leu Gln Ser Phe Arg Gln Asp Val Asp Asn Ala Ser Leu Ala Arg
   1               5                   10                  15
<210> 3
   <211> 14
   <212> PRT
   <213> Homo sapiens
<400> 3
   Leu Gln Ser Phe Arg Gln Asp Val Asp Asn Ala Ser Leu Ala
   1               5                   10
<210> 4
   <211> 14
   <212> PRT
   <213> Homo sapiens
<400> 4
   Thr Leu Gln Ser Phe Arg Gln Asp Val Asp Asn Ala Ser Leu
   1               5                   10
<210> 5
   <211> 18
   <212> PRT
   <213> Homo sapiens
<400> 5
   Asn Asp Lys Ala Arg Val Glu Val Glu Arg Asp Asn Leu Ala Glu Asp
   1               5                   10                  15
   Ile Met
<210> 6
   <211> 17
   <212> PRT
   <213> Homo sapiens
<400> 6
   Asp Lys Ala Arg Val Glu Val Glu Arg Asp Asn Leu Ala Glu Asp Ile
   1               5                   10                  15
   Met
<210> 7
   <211> 16
   <212> PRT
   <213> Homo sapiens
<400> 7
   Ser Pro Lys Tyr Ile Lys Met Phe Val Leu Asp Glu Ala Asp Glu Met
   1               5                   10                  15
<210> 8
   <211> 15
   <212> PRT
   <213> Homo sapiens
<400> 8
   Ser Pro Lys Tyr Ile Lys Met Phe Val Leu Asp Glu Ala Asp Glu
   1               5                   10                  15
<210> 9
   <211> 18
   <212> PRT
   <213> Homo sapiens
<400> 9
   Gly Ser Ser Arg Val Leu Ile Thr Thr Asp Leu Leu Ala Arg Gly Ile
   1               5                   10                  15
   Asp Val
<210> 10
   <211> 14
   <212> PRT
   <213> Homo sapiens
<400> 10
   Lys Ser Lys Ile Glu Asp Ile Arg Ala Glu Gln Glu Arg Glu
   1               5                   10
<210> 11
   <211> 13
   <212> PRT
   <213> Homo sapiens
<400> 11
   Lys Ser Lys Ile Glu Asp Ile Arg Ala Glu Gln Glu Arg
   1               5                   10
<210> 12
   <211> 17
   <212> PRT
   <213> Homo sapiens
<400> 12
   Gly Gln Asp Leu Leu Phe Lys Asp Ala Thr Val Arg Ala Val Pro Val
   1               5                   10                  15
   Gly
<210> 13
   <211> 16
   <212> PRT
   <213> Homo sapiens
<400> 13
   Gly Gln Asp Leu Leu Phe Lys Asp Ala Thr Val Arg Ala Val Pro Val
   1               5                   10                  15
<210> 14
   <211> 20
   <212> PRT
   <213> Homo sapiens
<400> 14
   Cys Ala Gly Thr Gly Cys Gly Thr Gly Thr Cys Ala Gly Cys Cys Ala
   1               5                   10                  15
   Ala Gly Thr Cys
               20
<210> 15
   <211> 19
   <212> PRT
   <213> Homo sapiens
<400> 15
   Thr Thr Cys Cys Cys Cys Gly Cys Cys Gly Thr Gly Thr Ala Ala Ala
   1               5                   10                  15
   Thr Gly Thr
<210> 16
   <211> 22
   <212> PRT
   <213> Homo sapiens
<400> 16
   Ala Gly Cys Gly Thr Cys Gly Ala Cys Cys Thr Gly Cys Thr Cys Ala
   1               5                   10                  15
   Gly Cys Cys Thr Gly Gly
               20
<210> 17
   <211> 24
   <212> PRT
   <213> Homo sapiens
<400> 17
   Leu Pro Lys Pro Pro Lys Pro Val Ser Lys Met Arg Met Ala Thr Pro
   1               5                   10                  15
   Leu Leu Met Gln Ala Leu Pro Met
               20
<210> 18
   <211> 9
   <212> PRT
   <213> Homo sapiens
<400> 18
   Phe Arg Gln Asp Val Asp Asn Ala Ser
   1               5
<210> 19
   <211> 9
   <212> PRT
   <213> Homo sapiens
<400> 19
   Val Glu Val Glu Arg Asp Asn Leu Ala
   1               5
<210> 20
   <211> 9
   <212> PRT
   <213> Homo sapiens
<400> 20
   Leu Phe Lys Asp Ala Thr Val Arg Ala
   1               5
<210> 21
   <211> 17
   <212> PRT
   <213> Homo sapiens
<400> 21
   Ala Pro Pro Ala Tyr Glu Lys Leu Ser Ala Glu Gln Ser Pro Pro Pro
   1               5                   10                  15
   Tyr
<210> 22
   <211> 738
   <212> PRT
   <213> Homo sapiens
<300>
   <308> swissprot/P08582
   <309> 2001-10-16
   <313> (1)..(738)
<400> 22
   Met Arg Gly Pro Ser Gly Ala Leu Trp Leu Leu Leu Ala Leu Arg Thr
   1               5                   10                  15
   Val Leu Gly Gly Met Glu Val Arg Trp Cys Ala Thr Ser Asp Pro Glu
               20                  25                  30
   Gln His Lys Cys Gly Asn Met Ser Glu Ala Phe Arg Glu Ala Gly Ile
           35                  40                  45
   Gln Pro Ser Leu Leu Cys Val Arg Gly Thr Ser Ala Asp His Cys Val
       50                  55                  60
   Gln Leu Ile Ala Ala Gln Glu Ala Asp Ala Ile Thr Leu Asp Gly Gly
   65                  70                  75                  80
   Ala Ile Tyr Glu Ala Gly Lys Glu His Gly Leu Lys Pro Val Val Gly
                   85                  90                  95
   Glu Val Tyr Asp Gln Glu Val Gly Thr Ser Tyr Tyr Ala Val Ala Val
               100                 105                 110
   Val Arg Arg Ser Ser His Val Thr Ile Asp Thr Leu Lys Gly Val Lys
           115                 120                 125
   Ser Cys His Thr Gly Ile Asn Arg Thr Val Gly Trp Asn Val Pro Val
       130                 135                 140
   Gly Tyr Leu Val Glu Ser Gly Arg Leu Ser Val Met Gly Cys Asp Val
   145                 150                 155                 160
   Leu Lys Ala Val Ser Asp Tyr Phe Gly Gly Ser Cys Val Pro Gly Ala
                   165                 170                 175
   Gly Glu Thr Ser Tyr Ser Glu Ser Leu Cys Arg Leu Cys Arg Gly Asp
               180                 185                 190
   Ser Ser Gly Glu Gly Val Cys Asp Lys Ser Pro Leu Glu Arg Tyr Tyr
           195                 200                 205
   Asp Tyr Ser Gly Ala Phe Arg Cys Leu Ala Glu Gly Ala Gly Asp Val
       210                 215                 220
   Ala Phe Val Lys His Ser Thr Val Leu Glu Asn Thr Asp Gly Lys Thr
   225                 230                 235                 240
   Leu Pro Ser Trp Gly Gln Ala Leu Leu Ser Gln Asp Phe Glu Leu Leu
                   245                 250                 255
   Cys Arg Asp Gly Ser Arg Ala Asp Val Thr Glu Trp Arg Gln Cys His
               260                 265                 270
   Leu Ala Arg Val Pro Ala His Ala Val Val Val Arg Ala Asp Thr Asp
           275                 280                 285
   Gly Gly Leu Ile Phe Arg Leu Leu Asn Glu Gly Gln Arg Leu Phe Ser
       290                 295                 300
   His Glu Gly Ser Ser Phe Gln Met Phe Ser Ser Glu Ala Tyr Gly Gln
   305                 310                 315                 320
   Lys Asp Leu Leu Phe Lys Asp Ser Thr Ser Glu Leu Val Pro Ile Ala
                   325                 330                 335
   Thr Gln Thr Tyr Glu Ala Trp Leu Gly His Glu Tyr Leu His Ala Met
               340                 345                 350
   Lys Gly Leu Leu Cys Asp Pro Asn Arg Leu Pro Pro Tyr Leu Arg Trp
           355                 360                 365
   Cys Val Leu Ser Thr Pro Glu Ile Gln Lys Cys Gly Asp Met Ala Val
   370 375 380
   Ala Phe Arg Arg Gln Arg Leu Lys Pro Glu Ile Gln Cys Val Ser Ala
   385 390 395 400
   Lys Ser Pro Gln His Cys Met Glu Arg Ile Gln Ala Glu Gln Val Asp
   405 410 415
   Ala Val Thr Leu Ser Gly Glu Asp Ile Tyr Thr Ala Gly Lys Lys Tyr
   420 425 430
   Gly Leu Val Pro Ala Ala Gly Glu His Tyr Ala Pro Glu Asp Ser Ser
   435 440 445
   Asn Ser Tyr Tyr Val Val Ala Val Val Arg Arg Asp Ser Ser His Ala
   450 455 460
   Phe Thr Leu Asp Glu Leu Arg Gly Lys Arg Ser Cys His Ala Gly Phe
   465 470 475 480
   Gly Ser Pro Ala Gly Trp Asp Val Pro Val Gly Ala Leu Ile Gln Arg
   485 490 495
   Gly Phe Ile Arg Pro Lys Asp Cys Asp Val Leu Thr Ala Val Ser Glu
   500 505 510
   Phe Phe Asn Ala Ser Cys Val Pro Val Asn Asn Pro Lys Asn Tyr Pro
   515 520 525
   Ser Ser Leu Cys Ala Leu Cys Val Gly Asp Glu Gln Gly Arg Asn Lys
   530 535 540
   Cys Val Gly Asn Ser Gln Glu Arg Tyr Tyr Gly Tyr Arg Gly Ala Phe
   545 550 555 560
   Arg Cys Leu Val Glu Asn Ala Gly Asp Val Ala Phe Val Arg His Thr
   565 570 575
   Thr Val Phe Asp Asn Thr Asn Gly His Asn Ser Glu Pro Trp Ala Ala
   580 585 590
   Glu Leu Arg Ser Glu Asp Tyr Glu Leu Leu Cys Pro Asn Gly Ala Arg
   595 600 605
   Ala Glu Val Ser Gln Phe Ala Ala Cys Asn Leu Ala Gln Ile Pro Pro
   610 615 620
   His Ala Val Met Val Arg Pro Asp Thr Asn Ile Phe Thr Val Tyr Gly
   625 630 635 640
   Leu Leu Asp Lys Ala Gln Asp Leu Phe Gly Asp Asp His Asn Lys Asn
   645 650 655
   Gly Phe Lys Met Phe Asp Ser Ser Asn Tyr His Gly Gln Asp Leu Leu
   660 665 670
   Phe Lys Asp Ala Thr Val Arg Ala Val Pro Val Gly Glu Lys Thr Thr
   675 680 685
   Tyr Arg Gly Trp Leu Gly Leu Asp Tyr Val Ala Ala Leu Glu Gly Met
       690                 695                 700
   Ser Ser Gln Gln Cys Ser Gly Ala Ala Ala Pro Ala Pro Gly Ala Pro
   705                 710                 715                 720
   Leu Leu Pro Leu Leu Leu Pro Ala Leu Ala Ala Arg Leu Leu Pro Pro
                   725                 730                 735
   Ala Leu